# EUROPEAN PATENT APPLICATION

(11) **EP 4 834 874 A1**
(43) Date of publication of application: **04.11.2026**
(21) Application number: 24912727.5
(22) Date of filing: 23.12.2024
(51) Int. Cl.: C07C 43/225, H10K 50/82, H10K 50/826, H10K 50/828, H10K 71/20, H10K 71/60, H10K 102/20

(54) **COMPOUND, CONDUCTIVE FILM, PHOTOELECTRONIC DEVICE, AND METHOD FOR PRODUCING CONDUCTIVE FILM**

(30) Priority: 27.12.2023 JP 2023220240
(71) Applicant: AGC Inc., Chiyoda-ku Tokyo 100-8405 (JP)
(72) Inventor: SUZUKI, Haruya, Tokyo 100-8405 (JP); KAMITANI, Ryosuke, Tokyo 100-8405 (JP); TSURUOKA, Kaori, Tokyo 100-8405 (JP); NAKAJIMA, Sho, Tokyo 100-8405 (JP)
(74) Representative: Müller-Boré & Partner Patentanwälte PartG mbB
(86) International application number: PCT/JP2024/045465
(87) International publication number: WO 2025/142840

(57) **Abstract**

A compound having a molecular weight within a range from 500 to 5,000, composed of hydrogen atoms, carbon atoms and hetero atoms, wherein the hetero atoms include at least one type of hetero atom selected from among an oxygen atom, sulfur atom, fluorine atom and silicon atom, and having at least one alicyclic structure, at least one aromatic ring structure bonded directly to the alicyclic structure, and at least one monovalent substituent Ms bonded directly to the aromatic ring structure, wherein the monovalent substituent Ms is at least one type of group selected from among monovalent groups containing a fluorine-containing aliphatic group, monovalent groups containing a fluorinated sulfur group, monovalent groups containing a siloxanyl group, and monovalent groups containing a saturated hydrocarbon group.

## Description

### [Technical Field]

The present disclosure relates to a compound, a conductive film, a photoelectronic device, and a method for producing a conductive film. Priority is claimed on Japanese Patent Application No. 2023-220240, filed December 27, 2023, the content of which is incorporated herein by reference.

### [Background Art]

Conventionally, conductive films having two types of regions with different light transmittances are well known as the conductive films for photoelectronic devices such as organic electroluminescent elements (hereinafter also referred to as "organic EL elements").

In the following explanation, of the two types of regions with different light transmittances, a region having a relatively lower light transmittance is referred to as a "first region," and a region with a higher light transmittance is referred to as a "second region."

In recent years, one known method for producing the types of conductive films described above is a method that uses an organic material that has a characteristic of repelling metal (hereinafter also referred to as "metal patterning material"). In this method, a pattern film is formed on a substrate using the metal patterning material, and a metal is then deposited on top of this pattern film. At this time, in each region where the pattern film has been formed, either no metal is deposited at all, or only a small amount of metal is deposited, forming a second region with high light transmittance. In contrast, in each region where the pattern film has not been formed, a large amount of metal is deposited, forming a first region with relatively low light transmittance.

Patent Document 1 discloses a compound that can be used as a metal patterning material for deposition, the compound having a specific aromatic ring and/or heteroaromatic ring, a fluorine atom and at least one tertiary amine within the molecule, having no condensed aromatic rings of 16 or more carbon atoms, having a molecular weight within a range from 500 to 3,000, and having a glass transition temperature of 60°C or higher.

### [Citation List]

### [Patent Document]

[Patent Document 1] International Patent Publication No. 2022/034907

### [Summary of Invention]

### [Technical Problem]

However, the compound disclosed in Patent Document 1 sometimes exhibits insufficient silver (Ag) repellency. For example, when Ag is deposited on top of a pattern film formed from the compound disclosed in Patent Document 1, Ag may adhere to the surface of the pattern film, and even if the second region appears transparent, the visible light transmittance, and particularly the blue light transmittance, may sometimes be lower than before the Ag deposition.

The present disclosure provides a compound having excellent Ag repellency, and also provides a conductive film and a photoelectronic device formed using this compound, and a method for producing a conductive film.

### [Solution to Problem]

The present disclosure provides a compound, a conductive film, a photoelectronic device, and a method for producing a conductive film having the following aspects [1] to [12].
[1] A compound having a molecular weight within a range from 500 to 5,000,
   composed of hydrogen atoms, carbon atoms and hetero atoms, wherein the hetero atoms include at least one type of hetero atom selected from among an oxygen atom, sulfur atom, fluorine atom and silicon atom, and
   having at least one alicyclic structure, at least one aromatic ring structure bonded directly to the alicyclic structure, and at least one monovalent substituent Ms bonded directly to the aromatic ring structure, wherein
   the monovalent substituent Ms is at least one type of group selected from among monovalent groups containing a fluorine-containing aliphatic group, monovalent groups containing a fluorinated sulfur group, monovalent groups containing a siloxanyl group, and monovalent groups containing a saturated hydrocarbon group.
[2] The compound according to [1], wherein the number of the alicyclic structures is two or more.
[3] The compound according to [1] or [2], wherein the alicyclic structure is monocyclic.
[4] The compound according to any one of [1] to [3], wherein the monovalent group containing a fluorine-containing aliphatic group is represented by formula f1 shown below:

   -Z¹-(O-R¹)ₙ-F Formula f1

   wherein R¹ represents an alkylene group or a fluoroalkylene group, n represents an integer of 1 or greater, the R¹ groups are the same or different, and Z¹ represents a single bond or a divalent linking group that does not contain an aromatic ring structure.
[5] The compound according to any one of [1] to [3], wherein the monovalent group containing a fluorine-containing aliphatic group is at least one group selected from among groups represented by formula f5 and formula f6 shown below:

   -Z²-(-X¹-Rf¹)ₙ₁ Formula f5

   -Z³-(-X²-Rf²-R²)n₂ Formula f6

   wherein Rf¹ represents -CF₃, -CF₂H or -CFH₂, Rf² represents -CF₂- or -CFH-, R² represents an alkyl group, X¹ represents -O- or -S-, Z² and Z³ each represent a single bond, or a (n1+1)-valent or (n2+1)-valent linking group that does not contain an aromatic ring structure, and n1 and n2 each represent an integer within a range from 1 to 9.
[6] The compound according to any one of [1] to [3], wherein the monovalent group containing a fluorinated sulfur group is at least one group selected from among groups represented by formula fs1 and formula fs2 shown below:

   -Z⁴-SF₅ Formula fs1

   -Z⁵-SF₄-R³ Formula fs2

   wherein R³ represents a hydrocarbon group, and Z⁴ and Z⁵ each represent a single bond or a divalent linking group that does not contain an aromatic ring structure.
[7] The compound according to any one of [1] to [3], wherein the monovalent group containing a siloxanyl group is at least one group selected from among groups represented by formula sx1 and formula sx2 shown below: 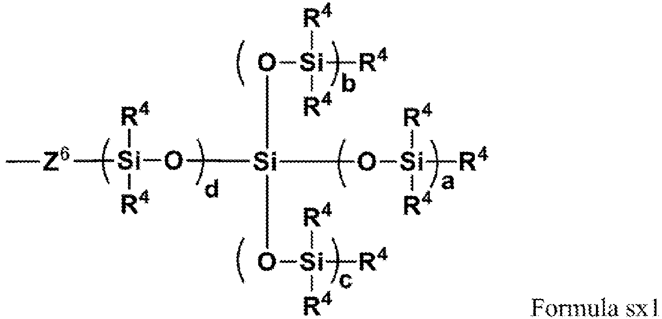 wherein each R⁴ independently represents an alkyl group of 1 to 10 carbon atoms or an aryl group of 6 to 12 carbon atoms, Z⁶ represents a single bond or a divalent linking group that does not contain an aromatic ring structure, and each of a, b, c and d independently represents an integer within a range from 0 to 20, provided that a+b+c+d > 0. 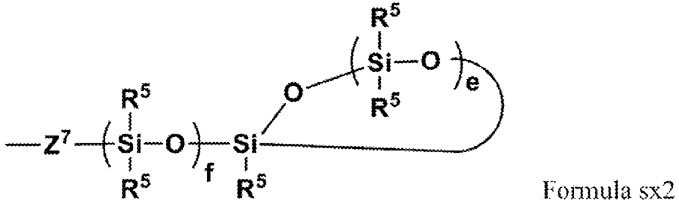 wherein each R⁵ independently represents an alkyl group of 1 to 10 carbon atoms or an aryl group of 6 to 12 carbon atoms, Z⁷ represents a single bond or a divalent linking group that does not contain an aromatic ring structure, e represents an integer within a range from 1 to 20, and f represents an integer within a range from 0 to 20, provided that e+f > 1.
[8] The compound according to any one of [1] to [3], wherein the monovalent group containing a saturated hydrocarbon group is represented by formula sh1 shown below:

   -Z⁸-R⁶ Formula sh1

   wherein R⁶ represents a saturated hydrocarbon group of 4 to 24 carbon atoms, and Z⁸ represents a single bond, a divalent linking group that does not contain an aromatic ring structure, an oxygen atom, or a sulfur atom.
[9] The compound according to any one of [1] to [8], represented by formula 1 shown below: 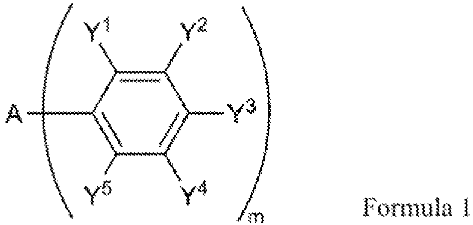 wherein m represents an integer within a range from 1 to 8, A represents an m-valent group containing the at least one alicyclic structure, Y¹ to Y⁵ each independently represent the monovalent substituent Ms, a hydrogen atom, a fluorine atom, or a monovalent aliphatic group having no fluorine atom (but excluding monovalent groups containing a saturated hydrocarbon group), and one or two of the combinations of Y¹ and Y², Y² and Y³, Y³ and Y⁴, and Y⁴ and Y⁵ are optionally bonded together to form a ring together with the carbon atoms bonded thereto, provided that at least one of Y¹ to Y⁵ represents the monovalent substituent Ms.
[10] The compound according to [9], wherein at least one of Y¹ to Y⁵ represents a monovalent group containing a fluorine-containing aliphatic group.
[11] The compound according to [10] or [11], wherein
   m in the formula 1 represents 2, and A represents a group represented by formula A-1a shown below, or
   m in the formula 1 represents 3, and A represents a group represented by formula A-2a shown below, or
   m in the formula 1 represents 4, and A represents a group represented by formula A-3a shown below: 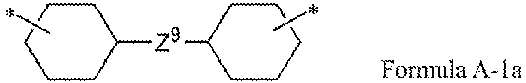 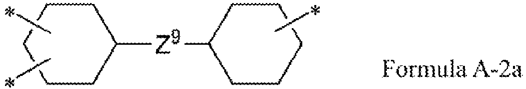 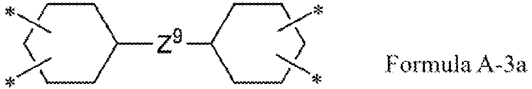 wherein Z⁹ represents a single bond or a divalent linking group, and * represents a bonding site.
[12] A conductive film having a first region, and a second region that exhibits a higher light transmittance than that of the first region, wherein
   the conductive film has a first film containing a conductive material, and a second film containing the compound according to any one of [1] to [11], and
   the first film is disposed so as to overlap at least the first region from among the first region and the second region, and the second film is disposed so as to overlap the second region.
[13] The conductive film according to [12], wherein the conductive material contains silver.
[14] A photoelectronic device having the conductive film according to [12] or [13].
[15] The photoelectronic device according to [14], having
   a substrate,
   an anode provided on the substrate,
   a cathode provided on the substrate, and
   an active layer disposed between the anode and the cathode, wherein
   the cathode is the conductive film.
[16] A method for producing a conductive film, the method including:
   depositing the compound according to any one of [1] to [11] on a substrate via a mask to form a second film containing the compound, and
   depositing a conductive material from above the second film.
[17] The method for producing a conductive film according to [16], wherein the conductive material contains silver.

### [Advantageous Effects of Invention]

The compound of the present disclosure exhibits excellent Ag repellency.

### [Brief Description of Drawings]

[FIG. 1] A plan view of a conductive film 1 according to an embodiment.
[FIG. 2] A cross-sectional view viewed in the direction of the arrows of the line II-II of FIG. 1.
[FIG. 3] A schematic diagram illustrating a method for producing the conductive film 1.
[FIG. 4] A schematic diagram illustrating a method for producing the conductive film 1.
[FIG. 5] A schematic diagram illustrating a method for producing the conductive film 1.
[FIG. 6] A schematic diagram illustrating a conductive film 2 according to an embodiment.
[FIG. 7] A schematic diagram illustrating a conductive film 3 according to an embodiment.
[FIG. 8] A cross-sectional schematic view illustrating a photoelectronic device (organic EL device) according to an embodiment.
[FIG. 9] A light transmittance spectrum across a wavelength range of 300 to 800 nm in Example 1.
[FIG. 10] A light transmittance spectrum across a wavelength range of 300 to 800 nm in Example 2.
[FIG. 11] A light transmittance spectrum across a wavelength range of 300 to 800 nm in Example 3.
[FIG. 12] A light transmittance spectrum across a wavelength range of 300 to 800 nm in Example 4.
[FIG. 13] A light transmittance spectrum across a wavelength range of 300 to 800 nm in Example 21.
[FIG. 14] A light transmittance spectrum across a wavelength range of 300 to 800 nm in Example 22.
[FIG. 15] A light transmittance spectrum across a wavelength range of 300 to 800 nm in Example 23.
[FIG. 16] A light transmittance spectrum across a wavelength range of 300 to 800 nm in Example 24.
[FIG. 17] A light transmittance spectrum across a wavelength range of 300 to 800 nm in Example 25.

### [Description of Embodiments]

Meanings and definitions of terms used in this description are as follows.

An "alkylene group" means a divalent saturated hydrocarbon group. A "fluoroalkylene group" means a group in which one or more hydrogen atoms of an alkylene group have each been substituted with a fluorine atom. A "polyfluoroalkylene group" means a group in which two or more hydrogen atoms of an alkylene group have each been substituted with a fluorine atom. A "perfluoroalkylene group" means a group in which all of the hydrogen atoms of an alkylene group have been substituted with fluorine atoms.

An "alkyl group" means a monovalent saturated hydrocarbon group.

An "etheric oxygen atom" means an oxygen atom that forms an ether bond (-O-) between carbon atoms.

In this description, compounds or groups represented by chemical formulas are also referred to with the number of the formula appended to the term "compound" or "group". For example, a compound represented by formula 1 is also referred to as "compound 1", and a group represented by formula f1 is also referred to as "group f1."

In this description, an expression "a to b" used to indicate a numerical range means a range that includes the numerical values before and after the "to" as the lower limit and upper limit respectively.

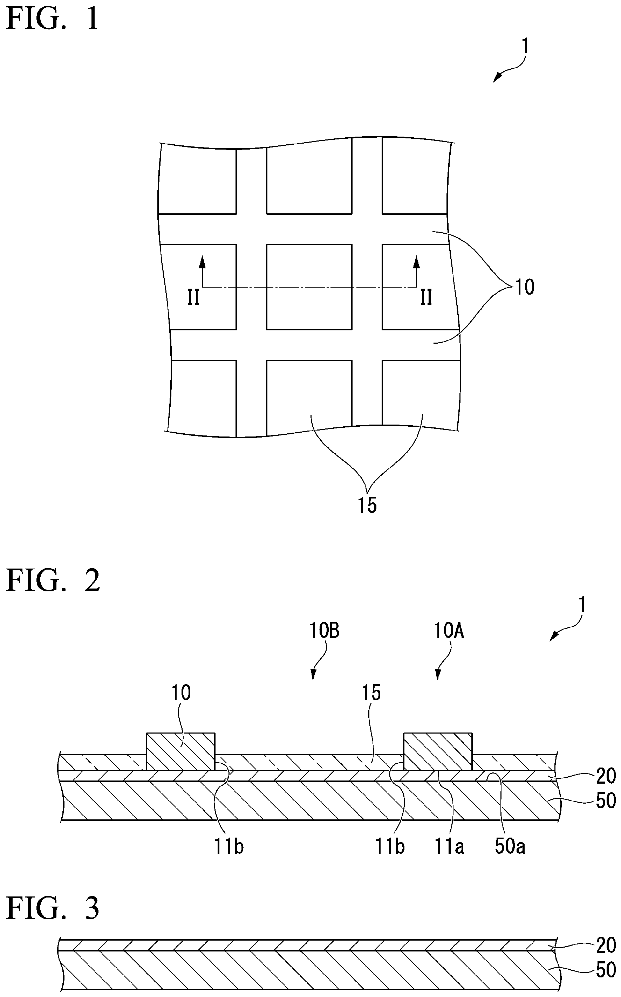

In FIGS. 1 to 8, the dimensions and proportions and the like of each component have been altered as appropriate in order to improve the clarity of the drawings.

### [Compound]

The compound of the present disclosure has a molecular weight within a range from 500 to 5,000, and is composed of hydrogen atoms, carbon atoms and hetero atoms. The hetero atoms include at least one type of hetero atom selected from among an oxygen atom, sulfur atom, fluorine atom and silicon atom. Moreover, the compound of the present disclosure has at least one alicyclic structure, at least one aromatic ring structure bonded directly to the alicyclic structure, and at least one monovalent substituent Ms bonded directly to the aromatic ring structure. The monovalent substituent Ms is at least one type of group selected from among monovalent groups containing a fluorine-containing aliphatic group, monovalent groups containing a fluorinated sulfur group, monovalent groups containing a siloxanyl group, and monovalent groups containing a saturated hydrocarbon group.

The hetero atom is included in at least one moiety selected from the group consisting of the alicyclic structure, the aromatic ring structure, and the substituent Ms, is preferably included in at least the substituent Ms, and is more preferably included in only the substituent Ms.

A substituent other than the aromatic ring structure may also be bonded to the alicyclic structure. This substituent other than the aromatic ring structure may be any substituent, provided the substituent does not contain atoms besides hydrogen atoms, carbon atoms and the hetero atoms, and examples include monovalent fluorine-containing aliphatic groups, monovalent aliphatic groups having no fluorine atoms (hereinafter also referred to as "fluorine-free aliphatic groups"), and a fluorine atom. The alicyclic structure, the monovalent fluorine-containing aliphatic groups, and the monovalent fluorine-free aliphatic groups are described below in further detail.

A substituent other than the alicyclic structure and the monovalent substituent Ms may also be bonded to the aromatic ring structure. This substituent other than the alicyclic structure and the monovalent substituent Ms may be any substituent, provided the substituent does not contain atoms besides hydrogen atoms, carbon atoms and the hetero atoms, and examples include monovalent fluorine-free aliphatic groups and a fluorine atom. The aromatic ring structure and the monovalent substituent Ms are described below in further detail.

### (Molecular Weight)

The molecular weight of the compound is within a range from 500 to 5,000, and is preferably from 600 to 3,000, more preferably from 600 to 2,000, and particularly preferably from 1,000 to 2,000. Provided the molecular weight of the compound is not more than the above upper limit, deposition can be conducted at a temperature below the thermal decomposition temperature. Provided the molecular weight of the compound is at least as large as the above lower limit, the film formability during deposition is excellent.

The molecular weight of the compound can be calculated by determining the structure of the compound by ¹H-NMR and/or ¹⁹F-NMR.

### (95% Mass Loss Temperature)

When the compound is heated from 50°C to 500°C at a rate of temperature increase of 2°C per minute under a degree of vacuum of not more than 1×10⁻³ Pa, the temperature at which the heat mass loss reaches 95% (hereinafter also referred to as the "95% mass loss temperature") is preferably within a range from 150 to 300°C. Provided the 95% mass loss temperature falls within this range, the deposition properties are excellent.

Details regarding the method used for measuring the heat mass loss temperature are as described below in the examples.

When the compound is deposited on the surface of a quartz substrate in an amount sufficient to form a film thickness of 50 nm, the visible light transmittance of the thus formed substrate with an organic film is preferably at least 85%, and more preferably 90% or higher. Provided the visible light transmittance is at least as high as the above lower limit, then even in those cases where a thin film of the compound is formed on the second region of the conductive film, the light transmittance of the second region is still excellent.

Details regarding the method used for measuring the visible light transmittance of the substrate with an organic film are as described below in the examples.

The compound is preferably a solid at 25°C. Provided the compound is a solid at 25°C, the shape stability of the deposited film is excellent.

### (Alicyclic Structure)

An alicyclic structure is a cyclic structure that does not contain an aromatic ring. **In** the alicyclic structure, the ring skeleton may be formed solely from carbon atoms, or may be a hetero ring that includes one or more atoms other than carbon atoms in the ring skeleton. However, because the compound is composed of hydrogen atoms, carbon atoms and the aforementioned hetero atoms, the alicyclic structure does not contain any atoms other than hydrogen atoms, carbon atoms and the hetero atoms. Accordingly, examples of the hetero ring include rings in which the ring skeleton contains carbon atoms and an oxygen atom or sulfur atom.

The alicyclic structure may be a monocyclic structure or a condensed ring structure.

The number of members in the monocyclic alicyclic structure is preferably within a range from 3 to 11, more preferably from 4 to 10, and particularly preferably from 5 to 8.

The number of rings constituting the alicyclic structure in the case of a condensed ring structure is preferably within a range from 2 to 6, and more preferably from 2 to 4. The number of members in those rings is preferably within a range from 3 to 11, more preferably from 4 to 10, and particularly preferably from 5 to 8.

The alicyclic structure may be saturated or unsaturated. In terms of yielding superior heat resistance, a saturated structure is preferred.

Examples of the alicyclic structure include a cyclopropane ring, cyclobutane ring, cyclopentane ring, cyclohexane ring, cycloheptane ring, cyclooctane ring, cyclononane ring, cyclodecane ring, cyclopropene ring, cyclobutene ring, cyclohexene ring, cycloheptene ring, cyclooctene ring, norbornane ring, adamantane ring, bicycloundecane ring, decahydronaphthalene ring, cubane ring, spiropentadiene ring, decalin ring, bicyclo[3.3.0]octane ring, and bicyclo[2.2.2]octane ring. Among these, a cyclohexane ring, adamantane ring or bicyclo[3.3.0]octane ring is preferred, and a cyclohexane ring is particularly desirable.

### (Aromatic Ring Structure)

The aromatic ring structure is a cyclic structure containing an aromatic ring. In the aromatic ring structure, the ring skeleton may be formed solely from carbon atoms, or may be a hetero ring that includes one or more atoms other than carbon atoms in the ring skeleton. However, because the compound is composed of hydrogen atoms, carbon atoms and the aforementioned hetero atoms, the aromatic ring structure does not contain any atoms other than hydrogen atoms, carbon atoms and the hetero atoms. Accordingly, examples of the hetero ring include rings in which the ring skeleton contains carbon atoms and an oxygen atom or sulfur atom.

The aromatic ring structure may be a monocyclic structure or a condensed ring structure. In terms of obtaining superior visible light transmittance, a monocyclic structure is preferred.

The number of members in the monocyclic aromatic ring structure is preferably within a range from 5 to 10, and more preferably from 5 to 6.

In the case of a condensed ring aromatic ring structure, the structure may be composed of two or more condensed aromatic rings, or one or more aromatic rings condensed with one or more alicyclic rings. However, a monovalent fluorine-containing aliphatic group is bonded to at least the aromatic ring. The number of rings constituting the condensed ring aromatic ring structure is preferably within a range from 2 to 4, and more preferably from 2 to 3. The number of members in those rings is preferably within a range from 5 to 10, and more preferably from 5 to 6.

Examples of the aromatic ring structure include a benzene ring, naphthalene ring, anthracene ring, azulene ring, furan ring, thiophene ring, thienothiophene ring, benzofuran ring, and benzothiophene ring. Among these, a benzene ring or thiophene ring is preferred, and a benzene ring is particularly desirable.

### (Monovalent Substituent Ms)

### <Monovalent groups containing a fluorine-containing aliphatic group>

A fluorine-containing aliphatic group is an aliphatic group that has a fluorine atom. An aliphatic group is an organic group that does not have an aromatic ring structure. However, because the compound is composed of hydrogen atoms, carbon atoms and the aforementioned hetero atoms, the monovalent group containing a fluorine-containing aliphatic group does not contain any atoms other than hydrogen atoms, carbon atoms and the hetero atoms.

The monovalent group containing a fluorine-containing aliphatic group may be chain-like, cyclic, or a combination thereof. In terms of achieving particularly superior Ag repellency, a chain-like group is preferred. The chain-like fluorine-containing aliphatic group may be linear or branched, but is preferably linear.

Further, the total number of carbon atoms in the entire monovalent group containing a fluorine-containing aliphatic group is preferably within a range from 1 to 20, more preferably from 1 to 10, and even more preferably from 2 to 6.

Examples of the monovalent group containing a fluorine-containing aliphatic group include fluoroalkyl ether groups, fluoroalkyl thioether groups, and fluoroalkyl groups. Examples of the fluoroalkyl ether groups include groups f1, f2 and f3 shown below. Examples of the fluoroalkyl groups include group f4 shown below. Among these, in terms of yielding particularly superior Ag repellency, fluoroalkyl ether groups are preferred, and the group f1 is particularly desirable.

-Z¹-(O-R¹)ₙ-F Formula f1

In this formula, R¹ represents an alkylene group or a fluoroalkylene group, n represents an integer of 1 or greater, the R¹ groups may be the same or different, and Z¹ represents a single bond or a divalent linking group that does not contain an aromatic ring structure.

-Z^{1a}-O-R^{f}-R^{1a}-F Formula f2

In this formula, R^{1a} represents a single bond, an alkylene group or a fluoroalkylene group, R^{f} represents a chain-like alkylene group or fluoroalkylene group, and Z^{1a} represents a single bond or a divalent linking group that does not contain an aromatic ring structure.

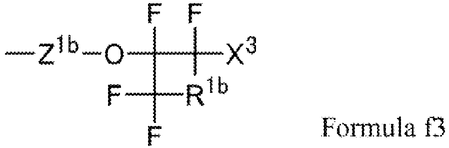

In this formula, X³ represents a hydrogen atom or a fluorine atom, R^{1b} represents an alkylene group or fluoroalkylene group that may have an etheric oxygen atom at a terminal, or a group in which an alkylene group or fluoroalkylene group of 2 or more carbon atoms that may have an etheric oxygen atom at a terminal has an etheric oxygen atom between carbon atoms of the group, and Z^{1b} represents a single bond or a divalent linking group that does not contain an aromatic ring structure.

-R^{1c}-F Formula f4

In this formula, R^{1c} represents an alkylene group or a fluoroalkylene group.

In formula f1, each alkylene group or fluoroalkylene group for R¹ may be chain-like, cyclic, or a combination thereof. In terms of achieving particularly superior Ag repellency, a chain-like (linear or branched) group is preferred, and a linear group is particularly desirable. The number of carbon atoms in each alkylene group or fluoroalkylene group is preferably not more than 4, more preferably not more than 2, and may be 1. A polyfluoroalkylene group is preferred as the fluoroalkylene group.

In formula f1, the R¹ groups may be the same or different.

In terms of achieving superior Ag repellency, among the R¹ groups, the R¹ group adjacent to the terminal F is preferably a polyfluoroalkylene group, and more preferably a perfluoroalkylene group.

In formula f1, n represents an integer of 1 or greater, and may be an integer of 2 or greater. In terms of achieving superior deposition on substrates, n is preferably not more than 4, more preferably not more than 3, and particularly preferably 2.

In formula f1, Z¹ represents a single bond or a divalent linking group that does not contain an aromatic ring structure. The divalent linking group may be any divalent group that does not contain an aromatic ring structure and does not contain any atoms other than hydrogen atoms, carbon atoms, and the aforementioned hetero atoms, and examples include alkylene groups and fluoroalkylene groups. Examples of these alkylene groups and fluoroalkylene groups include the same groups as those exemplified above for the alkylene group and fluoroalkylene group for R¹.

In terms of achieving particularly superior Ag repellency, Z¹ is preferably a single bond.

A group f1-1 is preferred as the group f1.

-Z¹-O-CF₂CFX^{3a}-(O-R^{1d})ₙ₋₁-F Formula f1-1

In this formula, R^{1d} represents an alkylene group or a fluoroalkylene group, and X^{3a} represents a hydrogen atom or a fluorine atom. Further, n and Z¹ are as described above.

Examples of R^{1d} include the same groups as those exemplified above for R¹. In terms of achieving particularly superior Ag repellency, R^{1d} is preferably a perfluoroalkylene group, and a linear perfluoroalkylene group of 1 to 4 carbon atoms is particularly desirable.

Specific examples of the group f1-1 include the groups shown below.

-O-CF₂CF₂-O-CF₂-F

-O-CF₂CHF-O-CF₂-F

-O-CF₂CF₂-O-(CF₂)₃-F

-O-CF₂CHF-O-(CF₂)₃-F

In formula f2, examples of the alkylene group and fluoroalkylene group for R^{1a} include the same groups as those exemplified above for the alkylene group and fluoroalkylene group for R¹.

The chain-like alkylene group or fluoroalkylene group for R^{f} may be linear or branched, and is preferably linear. The number of carbon atoms in R^{f} is preferably within a range from 1 to 4. A chain-like polyfluoroalkylene group is preferred as R^{f}.

Examples of Z^{1a} include the same groups as those exemplified above for Z¹.

A group f2-1 is preferred as the group f2.

-Z^{1a}-O-CF₂CFX^{3b}-R^{1a}-F Formula f2-1

In this formula, X^{3b} represents a hydrogen atom or a fluorine atom. R^{1a} and Z^{1a} are as described above.

In formula f3, examples of the alkylene group and fluoroalkylene group for R^{1b} include the same groups as those exemplified above for the alkylene group and fluoroalkylene group for R¹. In the case of a group having an etheric oxygen atom between the carbon atoms of an alkylene group or fluoroalkylene group of 2 or more carbon atoms, the number of these etheric oxygen atoms that exist between carbon atoms may be 1 or 2.

Examples of Z^{1b} include the same groups as those exemplified above for Z¹.

In formula f4, examples of R^{1c} include the same groups as those exemplified above for R¹.

Further, the above monovalent group containing a fluorine-containing aliphatic group may also be, for example, a group f5 or group f6 shown below.

-Z²(-X¹-Rf¹)ₙ₁ Formula f5

-Z³(-X²-Rf² R²)ₙ₂ Formula f6

In these formulas, Rf¹ represents -CF₃, -CF₂H or -CFH₂, Rf² represents -CF₂- or -CFH-, R² represents an alkyl group, X¹ and X² each represent -O- or -S-, Z² and Z³ each represent a single bond or a (n1+1)-valent or (n2+1)-valent linking group that does not contain an aromatic ring structure, and n1 and n2 each represent an integer within a range from 1 to 9. The (n1+1)-valent or (n2+1)-valent linking group may be any appropriate linking group that does not contain an aromatic ring structure and does not contain any atoms other than hydrogen atoms, carbon atoms, and the aforementioned hetero atoms, and examples include alkylene groups, alkylene ether groups, fluoroalkylene groups, and fluoroalkylene ether groups. These groups may be chain-like (linear or branched) groups, cyclic groups, or a combination thereof. Examples of the alkylene groups and fluoroalkylene groups include the same groups as those exemplified above for the alkylene group and fluoroalkylene group for R¹. In the alkylene ether groups and fluoroalkylene ether groups, the etheric oxygen atom may be positioned at the bonding terminal that bonds to the aromatic ring. The alkyl group for R² may be chain-like, cyclic, or a combination thereof. In terms of achieving particularly superior Ag repellency, a chain-like (linear or branched) group is preferred. The number of carbon atoms in the alkyl group is preferably not more than 10, more preferably not more than 4, and may be 1.

In terms of achieving particularly superior Ag repellency, n1 and n2 are each preferably within a range from 2 to 9, and from the viewpoint of ease of production, are preferably within a range from 1 to 3.

In terms of achieving particularly superior Ag repellency, Z² and Z³ preferably each represent a divalent to nonavalent linking group, and more preferably a tetravalent to nonavalent linking group, that does not contain an aromatic ring structure, whereas from the viewpoint of ease of production, Z² and Z³ preferably each represent a single bond or a divalent to tetravalent linking group that does not contain an aromatic ring structure, and more preferably each represent a single bond or a divalent linking group.

Specific examples of the group f5 include the groups shown below.

Namely, examples include -OCF₃, -SCF₃, -O-CH₂-OCF₃, -O-CH₂CH₂-OCF₃, - O-CH₂CH₂CH₂-OCF₃, -O-CH₂-SCF₃, -O-CH₂CH₂-SCF₃, -O-CH₂CH₂CH₂-SCF₃, -CH₂-OCF₃, -CH₂CH₂-OCF₃, -CH₂CH₂CH₂-OCF₃, and the groups shown below.

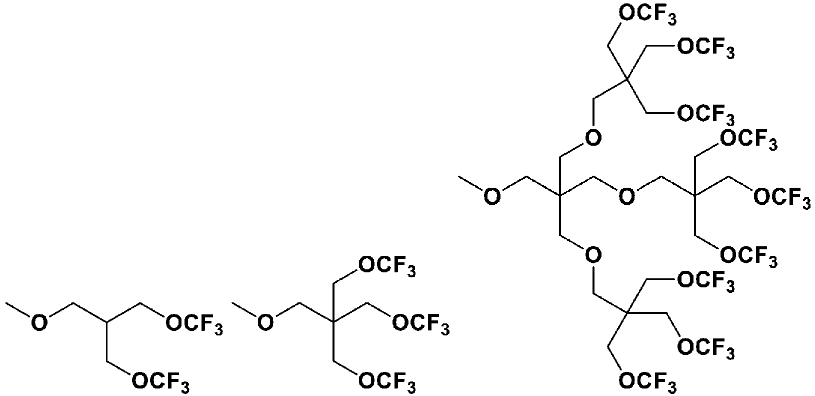

Specific examples of the group f6 include the groups shown below.

Namely, examples include -OCF₂H, -SCF₂H, -O-CH₂-OCF₂H, -O-CH₂CH₂-OCF₂H, -O-CH₂CH₂CH₂-OCF₂H, -O-CH₂-SCF₂H, -O-CH₂CH₂-SCF₂H, -O-CH₂CH₂CH₂-SCF₂H, -CH₂-OCF₂H, -CH₂CH₂-OCF₂H, -CH₂CH₂CH₂-OCF₂H, and the groups shown below.

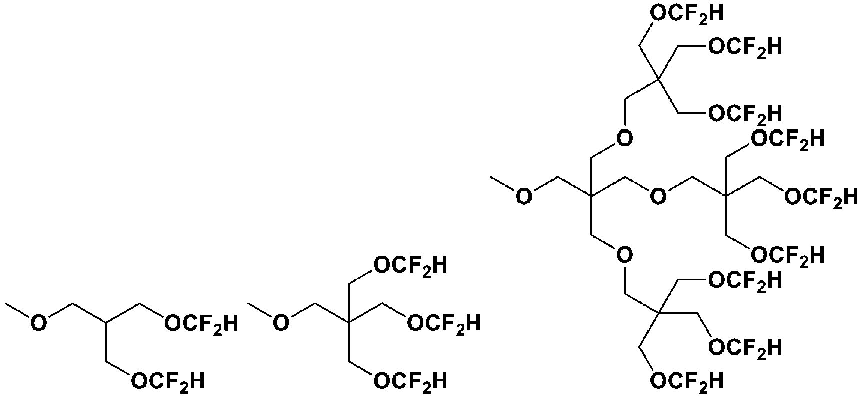

### <Monovalent groups containing a fluorinated sulfur group>

Examples of the monovalent group containing a fluorinated sulfur group include groups represented by formulas fs1 and fs2 shown below.

-Z⁴-SF₅ Formula fs1

-Z⁵-SF₄-R³ Formula fs2

In these formulas, R³ represents a hydrocarbon group, and Z⁴ and Z⁵ each represent a single bond or a divalent linking group that does not contain an aromatic ring structure.

The divalent linking group for Z⁴ and Z⁵ may be any divalent group that does not contain an aromatic ring structure and does not contain any atoms other than hydrogen atoms, carbon atoms, and the aforementioned hetero atoms, and examples include alkylene groups, alkylene ether groups, fluoroalkylene groups, and fluoroalkylene ether groups. Each of these groups may be chain-like (linear or branched), cyclic, or a combination thereof. Examples of the alkylene groups and fluoroalkylene groups include the same groups as those exemplified above for the alkylene group and fluoroalkylene group for R¹. In the alkylene ether groups and fluoroalkylene ether groups, the etheric oxygen atom may be positioned at the bonding terminal that bonds to the aromatic ring. In terms of achieving particularly superior Ag repellency, Z⁴ and Z⁵ are preferably single bonds.

Further, the number of carbon atoms in the entire monovalent group containing a fluorinated sulfur group is preferably within a range from 0 to 10, more preferably from 0 to 6, and even more preferably from 0 to 3.

### <Monovalent groups containing a siloxanyl group>

In this description, a siloxanyl group means a group having at least one Si-O-Si linkage. Examples of the monovalent group containing a siloxanyl group include groups represented by formulas sx1 and sx2 shown below.

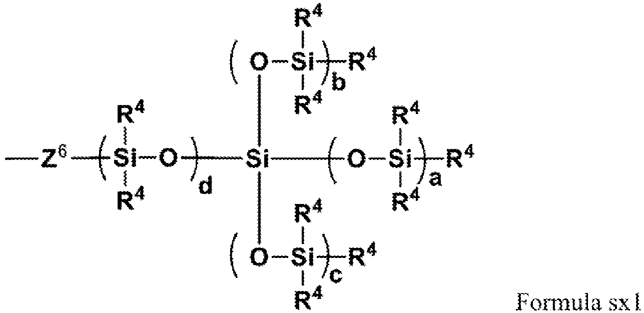

In the above formula, each R⁴ independently represents an alkyl group of 1 to 10 carbon atoms or an aryl group of 6 to 12 carbon atoms, Z⁶ represents a single bond or a divalent linking group that does not contain an aromatic ring structure, and each of a, b, c and d independently represents an integer within a range from 0 to 20, provided that a+b+c+d > 0. 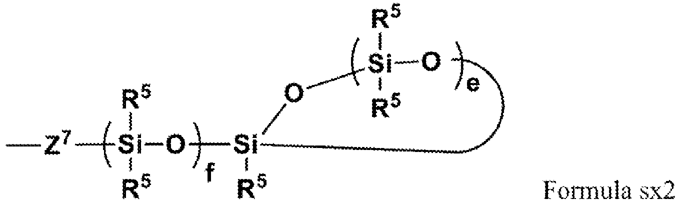 In the above formula, each R⁵ independently represents an alkyl group of 1 to 10 carbon atoms or an aryl group of 1 to 12 carbon atoms, Z⁷ represents a single bond or a divalent linking group that does not contain an aromatic ring structure, e represents an integer within a range from 1 to 20, and f represents an integer within a range from 0 to 20, provided that e+f > 1.

Specific examples of the alkyl group of 1 to 10 carbon atoms include a methyl group, ethyl group, propyl group, isopropyl group, butyl group, isobutyl group, sec-butyl group, t-butyl group, hexyl group, cyclohexyl group, 2-ethylhexyl group, and octyl group. Specific examples of the aryl group of 1 to 12 carbon atoms include a phenyl group and a naphthyl group. Among these, a methyl group is the most preferred.

The divalent linking group for Z⁶ and Z⁷ may be any divalent group that does not contain an aromatic ring structure and does not contain any atoms other than hydrogen atoms, carbon atoms, and the aforementioned hetero atoms, and examples include alkylene groups, alkylene ether groups, fluoroalkylene groups, and fluoroalkylene ether groups. Each of these groups may be chain-like (linear or branched), cyclic, or a combination thereof. Examples of the alkylene groups and fluoroalkylene groups include the same groups as those exemplified above for the alkylene group and fluoroalkylene group for R¹. In the alkylene ether groups and fluoroalkylene ether groups, the etheric oxygen atom may be positioned at the bonding terminal. In terms of achieving particularly superior Ag repellency, Z⁶ is preferably an ethylene group, trimethylene group, oxyethylene group, or oxytrimethylene group or the like.

Further, in formulas sx1 and sx2, the values of a+b+c+d and e+f are both preferably within a range from 1 to 20, more preferably from 2 to 10, and even more preferably from 2 to 4.

Examples of the group sx1 include the groups shown below.

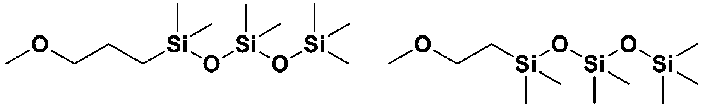

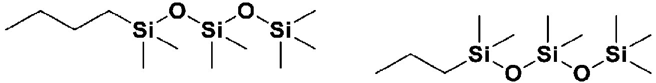

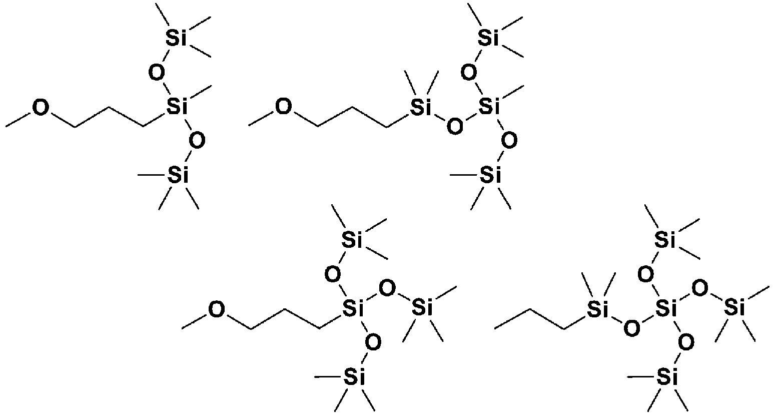

Examples of the group sx2 include the groups shown below.

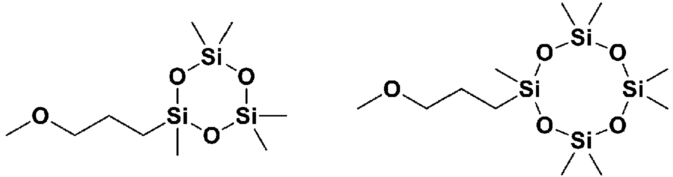

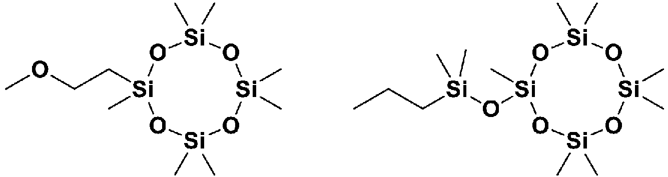

### <Monovalent groups containing a saturated hydrocarbon group>

Examples of the monovalent groups containing a saturated hydrocarbon group include groups represented by formula sh1 shown below.

-Z⁸-R⁶ Formula sh1

In this formula, R⁶ represents a saturated hydrocarbon group of 4 to 24 carbon atoms, and Z⁸ represents a single bond, a divalent linking group that does not contain an aromatic ring structure, an oxygen atom, or a sulfur atom. Examples of Z⁸ include the same groups as those exemplified above for Z¹.

The saturated hydrocarbon group for R⁶ is preferably a chain-like alkyl group of 12 to 24 carbon atoms, and is more preferably a chain-like alkyl group of 16 to 22 carbon atoms.

### (Monovalent groups containing fluorine-free aliphatic group)

Examples of the monovalent groups containing a fluorine-free aliphatic group include groups in which each of the fluorine atoms in one of the monovalent groups containing a fluorine-containing aliphatic group described above has been substituted with a hydrogen atom, and examples include alkyl ether groups and alkyl groups. These types of monovalent groups containing a fluorine-free aliphatic group enable adjustment of the molecular weight, boiling point, and glass transition temperature.

The monovalent fluorine-free aliphatic group is preferably an alkyl group. The alkyl group may be chain-like, cyclic, or a combination thereof. In terms of improving the molecular weight, the boiling point and the glass transition temperature, a methyl group, ethyl group, propyl group, isopropyl group, butyl group, isobutyl group, tert-butyl group, or cyclohexyl group is preferred, a methyl group, tert-butyl group or cyclohexyl group is more preferred, and a methyl group or tert-butyl group is particularly desirable.

The number of monovalent fluorine-free aliphatic group substituents on the aromatic ring is preferably within a range from 0 to 2, and is more preferably 0 or 1.

### (Structure of Compound)

In the compound, the number of alicyclic structures may be one, or two or more. This number of alicyclic structures may be set appropriately to ensure that the molecular weight of the compound falls within the range from 500 to 5,000. In terms of achieving particularly superior film formability during deposition, the number of alicyclic structures is preferably within a range from 2 to 6, and more preferably from 2 to 4.

In the compound, the number of aromatic ring structures bonded directly to a single alicyclic structure may be one, or two or more. In terms of achieving particularly superior film formability during deposition, 2 to 4 aromatic ring structures are preferred.

In those cases where the compound has two or more alicyclic structures, and two or more aromatic ring structures bonded directly to the alicyclic structures, the aromatic ring structures may be bonded to the same alicyclic structure, or bonded to different alicyclic structures.

In those cases where two or more aromatic ring structures are bonded to a single alicyclic structure, all of those aromatic ring structures may be bonded to different carbon atoms that constitute the ring skeleton of the alicyclic structure, or at least a portion of the aromatic ring structures may be bonded to the same carbon atom of the ring skeleton of the alicyclic structure. The maximum number of aromatic ring structures that can be bonded to a single carbon atom is two.

In the compound, the number of the monovalent substituents Ms bonded directly to a single aromatic ring structure may be one, or two or more. In terms of achieving particularly superior deposition properties, the number of monovalent substituents Ms is preferably not more than three, and is most preferably one.

There are no particular limitations on the bonding position of each monovalent substituent Ms in the aromatic ring structure, and the position may be selected appropriately with due consideration of factors such as ease of availability of the raw materials. In those cases where the aromatic ring structure is a benzene ring, the bonding position of the monovalent substituent Ms in the aromatic ring structure relative to the bonding position with the alicyclic structure is preferably the para-position.

One preferred example of the compound of the present disclosure is the compound 1 shown below. 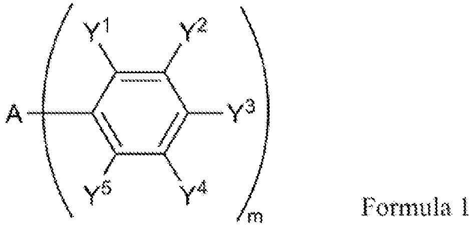 In the above formula, m represents an integer within a range from 1 to 8, A represents an m-valent group containing the at least one alicyclic structure, Y¹ to Y⁵ each independently represent the monovalent substituent Ms, a hydrogen atom, a fluorine atom, or a monovalent fluorine-free aliphatic group (but excluding monovalent groups containing a saturated hydrocarbon group), and one or two of the combinations of Y¹ and Y², Y² and Y³, Y³ and Y⁴, and Y⁴ and Y⁵ may be bonded together to form a ring together with the carbon atoms bonded thereto, provided that at least one of Y¹ to Y⁵ represents the monovalent substituent Ms. At least one of Y¹ to Y⁵ preferably represents an aforementioned monovalent group containing a fluorine-containing aliphatic group.

In terms of achieving superior film formability during deposition, m is preferably within a range from 2 to 6, more preferably from 2 to 4, and is most preferably 4.

The preferred range for the number of alicyclic structures contained within A is the same as the preferred range for the number of alicyclic structures within the compound.

Examples of A include groups composed of a single alicyclic structure, and groups in which two or more alicyclic structures are bonded together, either directly or via a linking group. Examples of groups in which two or more alicyclic structures are bonded together via a linking group include groups in which two alicyclic structures are bonded together via a single divalent linking group, groups in which a (wherein a is an integer of 3 or more) alicyclic structures are bonded together in a series arrangement via a-1 divalent linking groups, and groups in which a alicyclic structures are bonded together via a single a-valent linking group.

Examples of the divalent linking group include divalent hydrocarbon groups which may have a substituent. These divalent hydrocarbon groups may be chain-like, cyclic, or a combination thereof.

The divalent chain-like hydrocarbon groups may be saturated or unsaturated. The number of carbon atoms in these divalent chain-like hydrocarbon groups may be, for example, within a range from 1 to 6. A methylene group, which may have a substituent, is preferred as the divalent chain-like hydrocarbon group.

The divalent cyclic hydrocarbon groups may be alicyclic hydrocarbon groups or aromatic hydrocarbon groups. The alicyclic hydrocarbon groups may be saturated or unsaturated. The divalent cyclic hydrocarbon groups may be monocyclic or a condensed ring structure. The number of carbon atoms in these divalent cyclic hydrocarbon groups is, for example, typically within a range from 4 to 10.

The substituent that may be included in the divalent hydrocarbon group may be any group, provided the substituent does not contain atoms besides hydrogen atoms, carbon atoms, oxygen atoms and fluorine atoms, and specific examples include a fluorine atom and monovalent hydrocarbon groups which may have a substituent.

These monovalent hydrocarbon groups may be chain-like, cyclic, or a combination thereof.

The monovalent chain-like hydrocarbon groups may be saturated or unsaturated. The number of carbon atoms in these monovalent chain-like hydrocarbon groups is, for example, within a range from 1 to 6.

The monovalent cyclic hydrocarbon groups may be alicyclic hydrocarbon groups or aromatic hydrocarbon groups. The alicyclic hydrocarbon groups may be saturated or unsaturated. The monovalent cyclic hydrocarbon groups may be monocyclic or a condensed ring structure. The number of carbon atoms in these monovalent cyclic hydrocarbon groups is, for example, within a range from 4 to 10.

Examples of the substituent that may be included in the monovalent hydrocarbon group include the same groups as those exemplified above for the substituent that may be included in the divalent hydrocarbon group.

Examples of the a-valent linking group include groups in which a-2 hydrogen atoms have been removed from an aforementioned divalent hydrocarbon group which may have a substituent.

Examples of A in those cases where m represents 2 include groups A-1a, A-1b, A-1c and A-1d.

Examples of A in those cases where m represents 3 include groups A-2a, A-2b, A-2c and A-2d.

Examples of A in those cases where m represents 4 include groups A-3a, A-3b, A-3c and A-3d.

Among these groups, the groups A-3a, A-3b, A-3c and A-3d are preferred, and the group A-3a is particularly desirable.

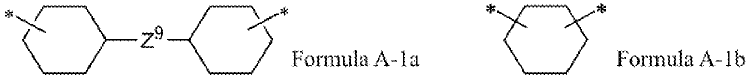

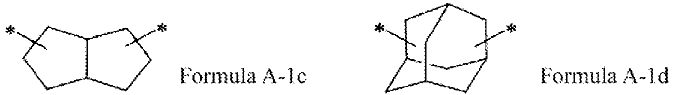

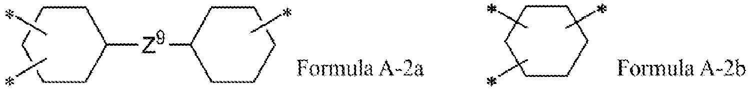

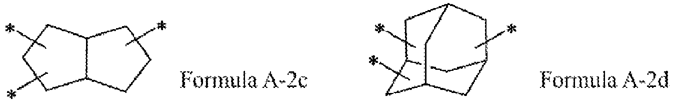

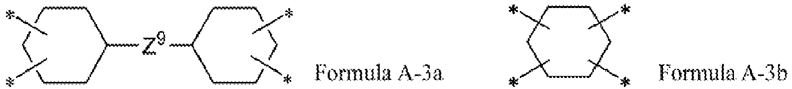

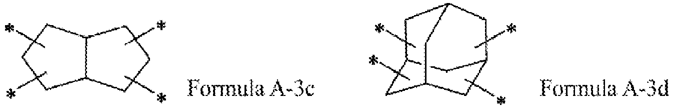

In the above formulas, Z⁹ represents a single bond or a divalent linking group, and * represents a bonding site.

In formula A-3a, the two bonding sites on each of the two cyclohexane rings may extend from the same carbon atom or from different carbon atoms among the carbon atoms that constitute the cyclohexane ring. In other words, among the carbon atoms that constitute the ring skeleton of each cyclohexane ring, two aromatic ring structures may be bonded to one carbon atom, or one aromatic ring structure may be bonded to each of two carbon atoms. In terms of achieving superior deposition properties, it is preferable that two aromatic ring structures are bonded to a single carbon atom. This also applies to one of the cyclohexane rings in formula A-2a. Furthermore, the same also applies to two bonding sites extending from the single cyclic structure in formulas A-1b, A-1d, A-2b, A-2c, A-2d, A-3b, A-3c, and A-3d.

A group A-3a-1 is preferred as the group A-3a.

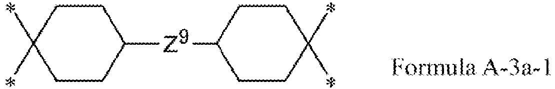

A group A-3b-1 is preferred as the group A-3b.

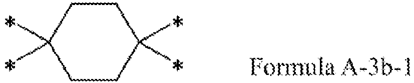

A group A-3c-1 is preferred as the group A-3c.

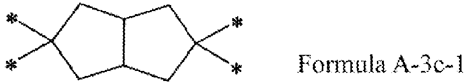

A group A-3d-1 is preferred as the group A-3d.

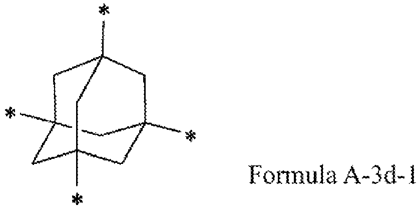

The group A-3a-1 may be, for example, a group A-3a-11 or a group A-3a-12.

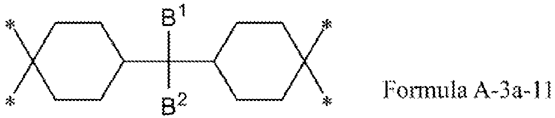

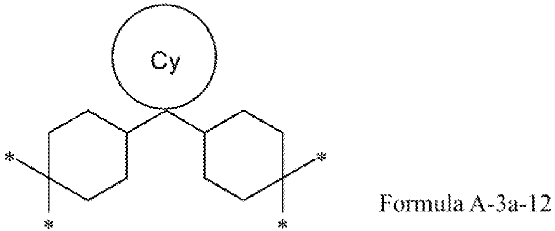

In the above formulas, B¹ and B² each represent a hydrogen atom, a fluorine atom, or a monovalent hydrocarbon group which may have a substituent, and Cy represents a divalent cyclic hydrocarbon group which may have a substituent.

In formula A-3a-11, examples of the monovalent hydrocarbon group for B¹ and B² include the same groups as those exemplified above for the monovalent hydrocarbon group which may have a substituent.

In formula A-3a-12, examples of Cy include the same groups as those exemplified above for the divalent cyclic hydrocarbon group for the aforementioned divalent linking group. The two cyclohexane rings in the formula are bonded to one carbon atom among the carbon atoms that constitute the Cy ring skeleton.

In formula 1, the monovalent substituent Ms and the monovalent fluorine-free aliphatic group for Y¹ to Y⁵ are the same as described above.

At least one of Y¹ to Y⁵ represents the monovalent substituent Ms. In terms of achieving superior deposition properties, it is preferable that only one of Y¹ to Y⁵ represents the monovalent substituent Ms, and more preferable that only Y³ represents the monovalent substituent Ms.

Examples of possible combinations of Y¹ to Y⁵ include the following.

Combination 1: Y³ represents a monovalent group containing a fluorine-containing aliphatic group, and Y¹, Y², Y⁴ and Y⁵ are hydrogen atoms.

Combination 2: Y³ represents a monovalent group containing a fluorine-containing aliphatic group, Y² represents a monovalent fluorine-free aliphatic group, and Y¹, Y⁴ and Y⁵ are hydrogen atoms.

Combination 3: Y³ represents a monovalent group containing a fluorine-containing aliphatic group, Y² and Y⁴ each represent a monovalent fluorine-free aliphatic group, and Y¹ and Y⁵ are hydrogen atoms.

Specific examples of the compound 1 include compounds 1-1 to 1-18 obtained in the examples described below, as well as the compounds shown below.

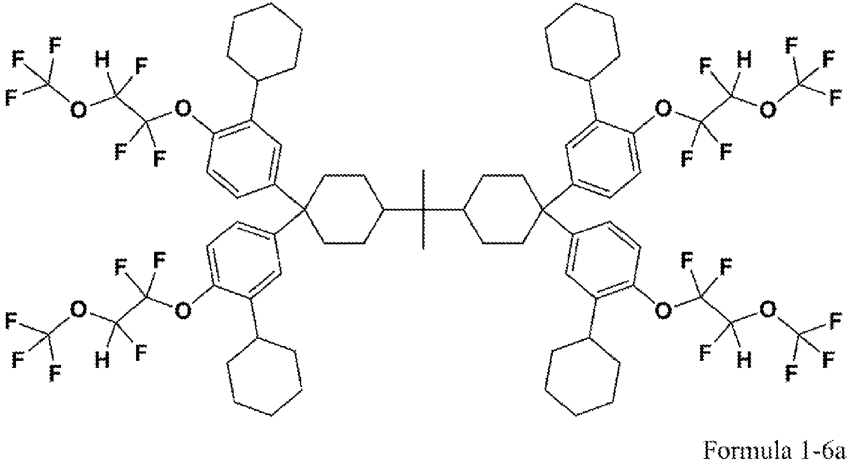

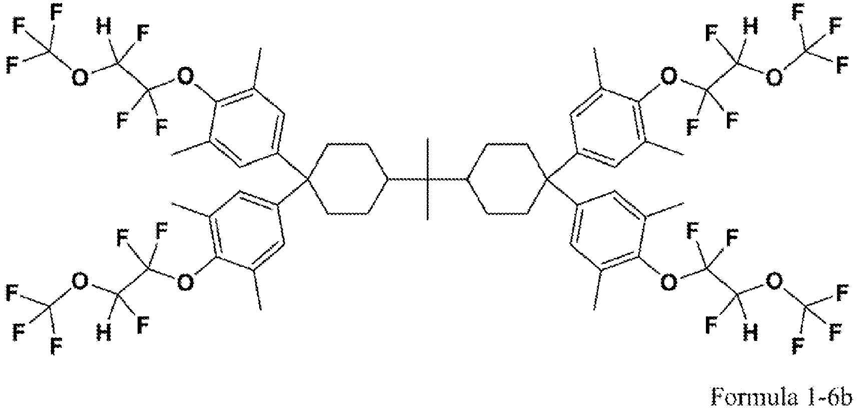

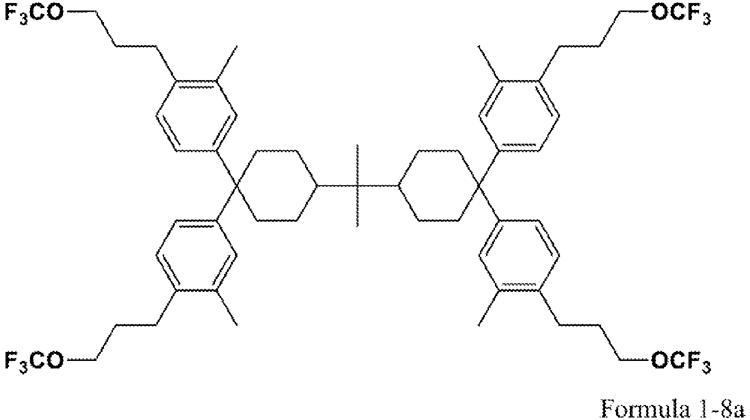

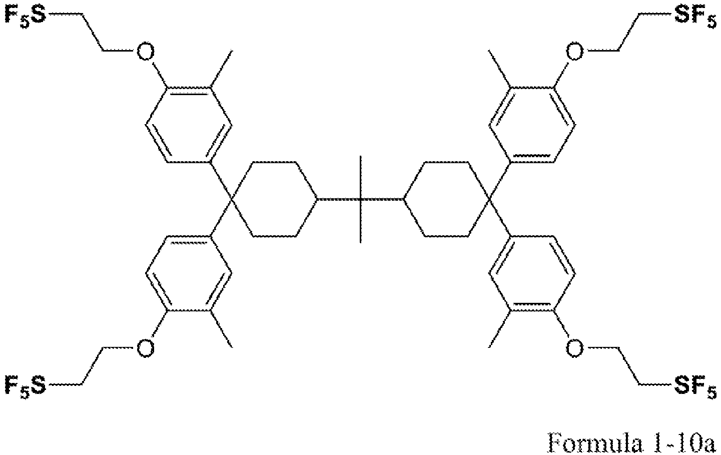

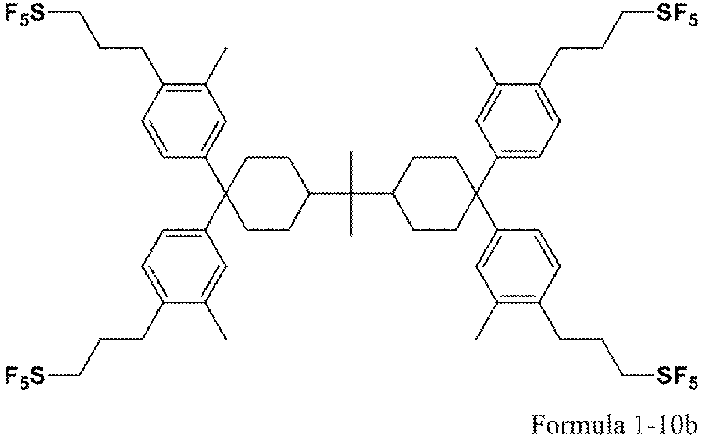

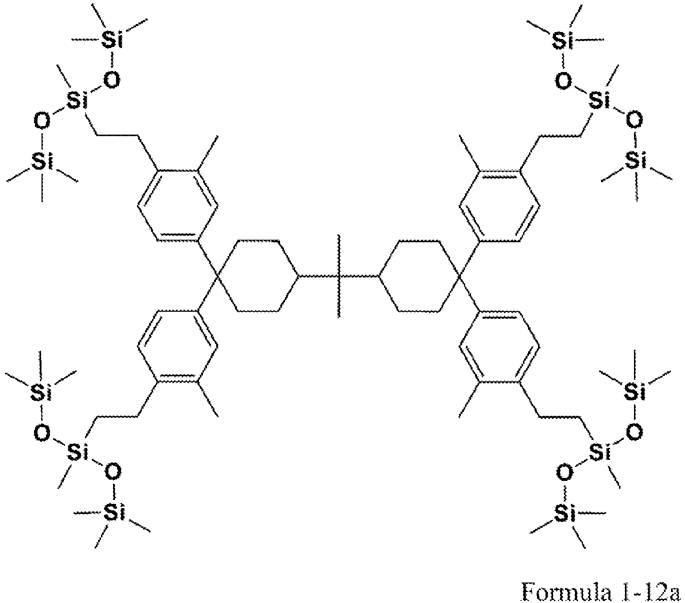

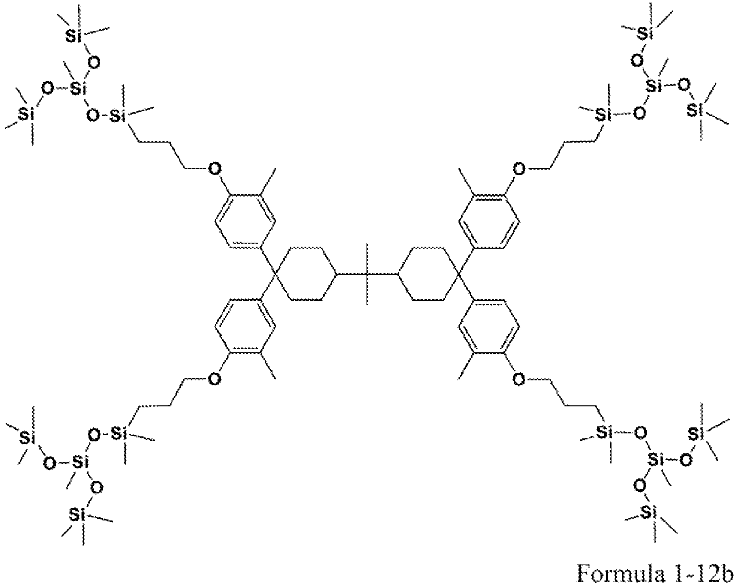

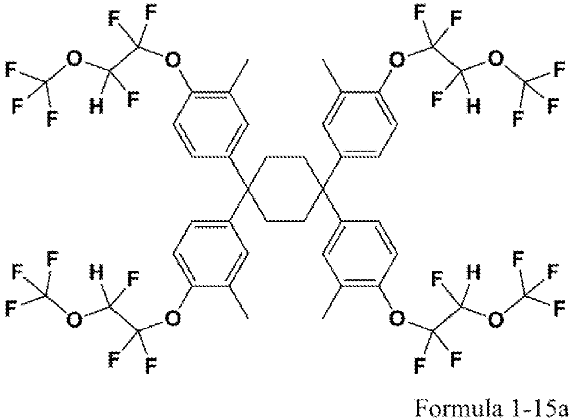

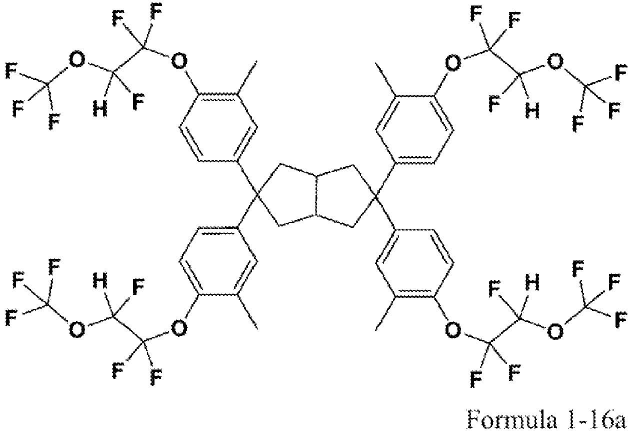

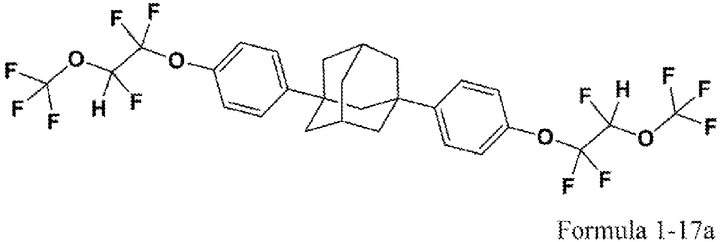

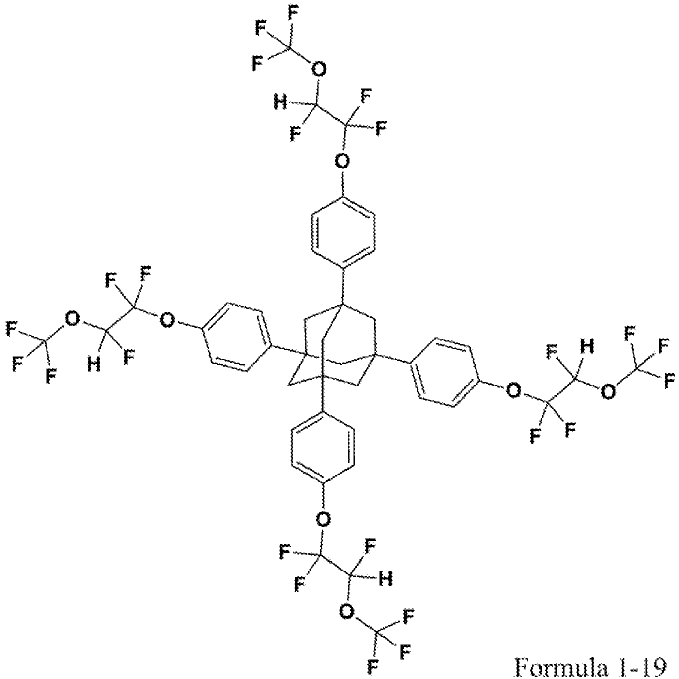

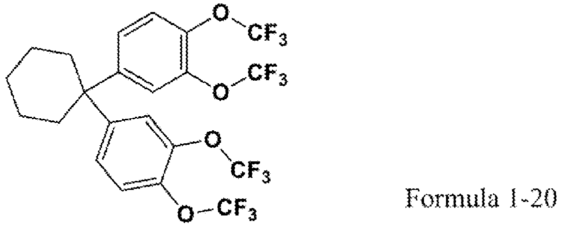

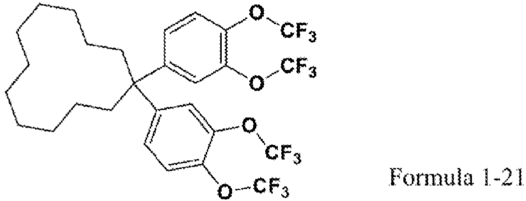

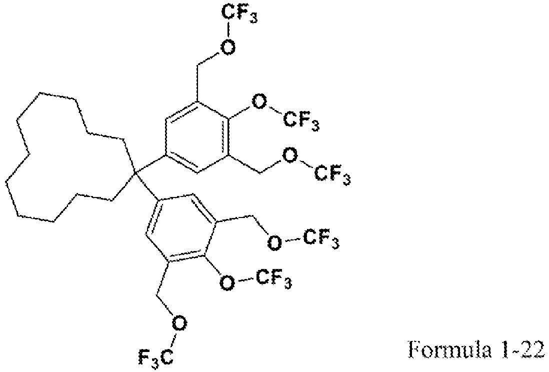

Preferred examples of the compound of the present disclosure include a compound 2.

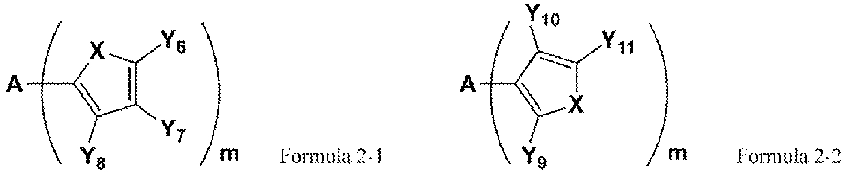

In the above formulas, X represents an oxygen atom or a sulfur atom, m represents an integer within a range from 1 to 8, A represents an m-valent group containing at least one alicyclic structure, Y⁶ to Y⁸ each independently represent an aforementioned monovalent substituent Ms, a hydrogen atom, a fluorine atom, or a monovalent fluorine-free aliphatic group (but excluding the aforementioned monovalent groups containing a saturated hydrocarbon group), either one or two of the combination of Y⁶ and Y⁷, and Y⁷ and Y⁸ may be bonded together to form a ring together with the carbon atoms bonded thereto, provided that at least one of Y⁶ to Y⁸ represents the monovalent substituent Ms. At least one of Y⁶ to Y⁸ preferably represents the aforementioned monovalent group containing a fluorine-containing aliphatic group. Each of Y⁹ to Y¹¹ independently represents an aforementioned monovalent substituent Ms, a hydrogen atom, a fluorine atom, or a monovalent fluorine-free aliphatic group (but excluding the aforementioned monovalent groups containing a saturated hydrocarbon group), the combination of Y¹⁰ and Y¹¹ may be bonded together to form a ring together with the carbon atoms bonded thereto, and at least one of Y⁹ to Y¹¹ represents the monovalent substituent Ms. At least one of Y⁹ to Y¹¹ preferably represents the aforementioned monovalent group containing a fluorine-containing aliphatic group.

In terms of achieving superior film formability during deposition, m is preferably within a range from 2 to 6, more preferably from 2 to 4, and most preferably 4.

The preferred range for the number of alicyclic structures contained within A is the same as the preferred range for the number of alicyclic structures within the compound.

Examples of A include groups composed of a single alicyclic structure, and groups in which two or more alicyclic structures are bonded together, either directly or via a linking group. Examples of groups in which two or more alicyclic structures are bonded together via a linking group include groups in which two alicyclic structures are bonded together via a single divalent linking group, groups in which a (wherein a is an integer of 3 or more) alicyclic structures are bonded together in a series arrangement via a-1 divalent linking groups, and groups in which a alicyclic structures are bonded together via a single a-valent linking group.

Examples of the divalent linking group include divalent hydrocarbon groups which may have a substituent. These divalent hydrocarbon groups may be chain-like, cyclic, or a combination thereof.

The divalent chain-like hydrocarbon groups may be saturated or unsaturated. The number of carbon atoms in these divalent chain-like hydrocarbon groups may be, for example, within a range from 1 to 6. A methylene group, which may have a substituent, is preferred as the divalent chain-like hydrocarbon group.

The divalent cyclic hydrocarbon groups may be alicyclic hydrocarbon groups or aromatic hydrocarbon groups. The alicyclic hydrocarbon groups may be saturated or unsaturated. The divalent cyclic hydrocarbon groups may be monocyclic or a condensed ring structure. The number of carbon atoms in these divalent cyclic hydrocarbon groups is, for example, typically within a range from 4 to 10.

The substituent that may be included in the divalent hydrocarbon group may be any group, provided the substituent does not contain atoms besides hydrogen atoms, carbon atoms, oxygen atoms and fluorine atoms, and specific examples include a fluorine atom and monovalent hydrocarbon groups which may have a substituent.

These monovalent hydrocarbon groups may be chain-like, cyclic, or a combination thereof.

The monovalent chain-like hydrocarbon groups may be saturated or unsaturated. The number of carbon atoms in these monovalent chain-like hydrocarbon groups is, for example, within a range from 1 to 6.

The monovalent cyclic hydrocarbon groups may be alicyclic hydrocarbon groups or aromatic hydrocarbon groups. The alicyclic hydrocarbon groups may be saturated or unsaturated. The monovalent cyclic hydrocarbon groups may be monocyclic or a condensed ring structure. The number of carbon atoms in these monovalent cyclic hydrocarbon groups is, for example, within a range from 4 to 10.

Examples of the substituent that may be included in the monovalent hydrocarbon group include the same groups as those exemplified above for the substituent that may be included in the divalent hydrocarbon group.

Examples of the a-valent linking group include linking groups in which a-2 hydrogen atoms have been removed from an aforementioned divalent hydrocarbon group which may have a substituent.

Examples of A include the same groups as those exemplified above in the case of the compound 1.

In formula 2-1 and formula 2-2, examples of the monovalent substituent Ms and the monovalent fluorine-free aliphatic group for Y⁶ to Y¹¹ include the same respective groups as those exemplified above.

At least one of Y⁶ to Y⁸ represents the monovalent substituent Ms. In terms of achieving superior deposition properties, it is preferable that only one of Y⁶ to Y⁸ represents the monovalent substituent Ms, and more preferable that only Y⁶ represents the monovalent substituent Ms.

At least one of Y⁹ to Y¹¹ represents the monovalent substituent Ms. In terms of achieving superior deposition properties, it is preferable that only one of Y⁹ to Y¹¹ represents the monovalent substituent Ms, and more preferable that only Y¹¹ represents the monovalent substituent Ms.

Examples of possible combinations of Y⁶ to Y⁹ include the following.

Combination 1: Y⁶ represents a monovalent group containing a fluorine-containing aliphatic group, and Y⁷ and Y⁸ are hydrogen atoms.

Combination 2: Y⁶ represents a monovalent group containing a fluorine-containing aliphatic group, Y⁷ represents a monovalent fluorine-free aliphatic group, and Y⁸ is a hydrogen atom.

Combination 3: Y⁶ represents a monovalent group containing a fluorine-containing aliphatic group, and Y⁷ and Y⁸ each represent a monovalent fluorine-free aliphatic group.

Specific examples of the compound 2 include compound 2-3 obtained **in** the examples described below, as well as the compounds shown below.

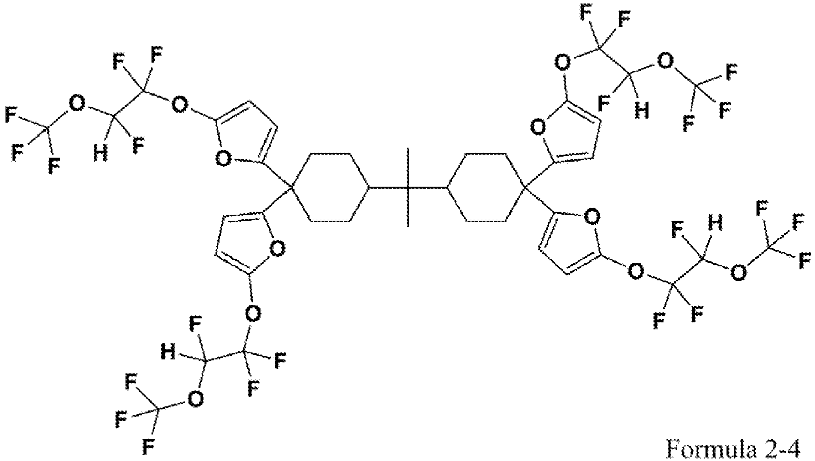

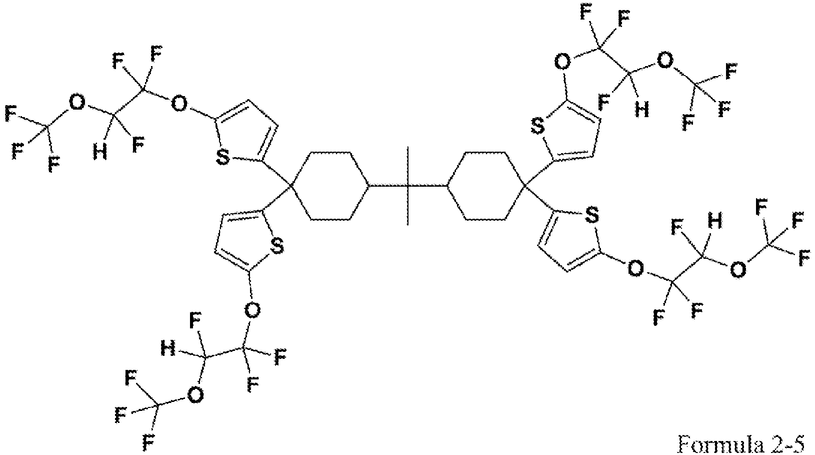

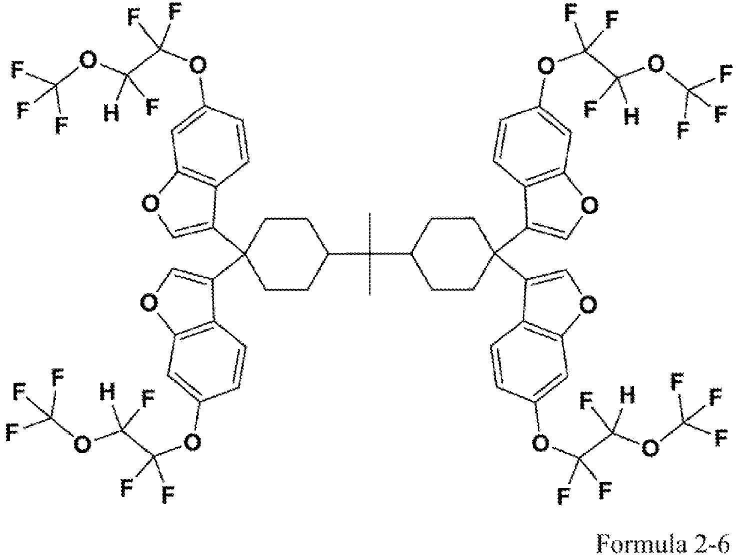

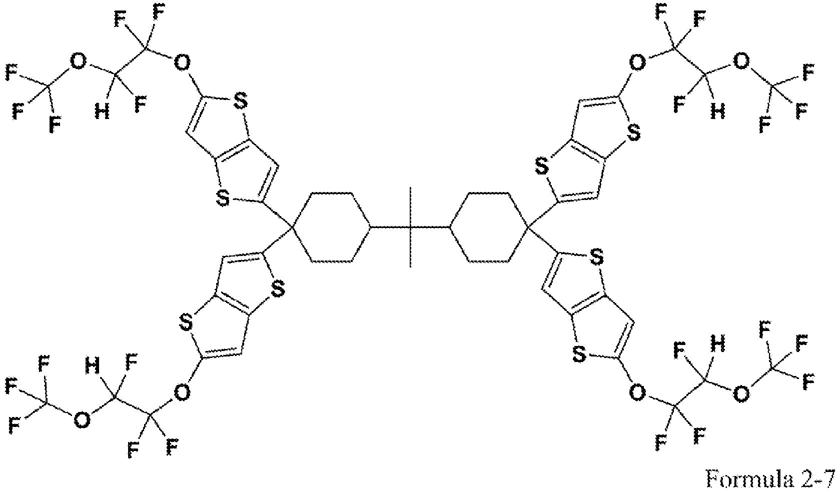

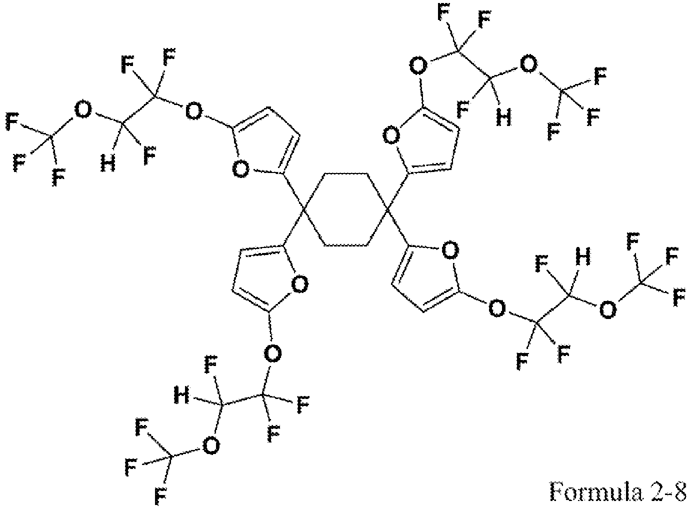

Other preferred examples of the compound of the present disclosure include a compound 3. 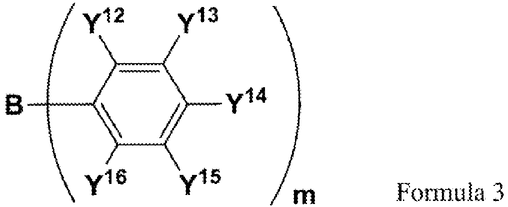 In the above formula, m represents an integer within a range from 1 to 8, B represents an m-valent linking group that does not contain an alicyclic structure, Y¹² to ^{y16} each independently represent an aforementioned monovalent substituent Ms, a hydrogen atom, a fluorine atom, a monovalent fluorine-free aliphatic group (but excluding the aforementioned monovalent groups containing a saturated hydrocarbon group), or a monovalent group containing at least one alicyclic structure, one or two of the combinations of Y¹² and Y¹³, Y¹³ and Y¹⁴, Y¹⁴ and Y¹⁵, and Y¹⁵ and Y¹⁶ may be bonded together to form a ring together with the carbon atoms bonded thereto, at least one of Y¹² to Y¹⁶ represents the monovalent substituent Ms, and at least one of Y¹² to Y¹⁶ represents an aforementioned monovalent group containing at least one alicyclic structure. At least one of Y¹² to Y¹⁶ preferably represents an aforementioned monovalent group containing a fluorine-containing aliphatic group.

In terms of achieving superior film formability during deposition, m is preferably within a range from 2 to 6, more preferably from 2 to 4, and most preferably 4.

The preferred range for the number of alicyclic structures contained within Y¹² to Y¹⁶ is from 1 to 2, and 1 is particularly preferred.

Specific examples of the compound 3 include compound 3-1 obtained in the examples described below.

### (Method for Producing Compound)

The compound of the present disclosure can be produced using appropriate combinations of conventional methods.

For example, the compound 1 having the group f1-1 as a monovalent group containing a fluorine-containing aliphatic group can be produced by reacting a compound p1 and a compound p2.

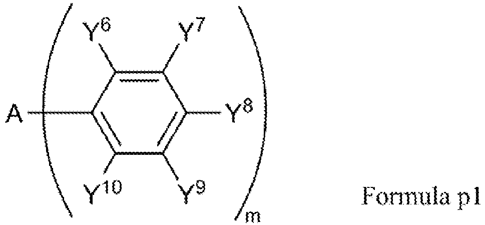

In the above formula, m and A are as described above, Y⁶ to Y¹⁰ each independently represent -Z^{1b}-OH, a hydrogen atom, a fluorine atom, or a monovalent aliphatic group that does not contain a fluorine atom, one or two of the combinations of Y⁶ and Y⁷, Y⁷ and Y⁸, Y⁸ and Y⁹, and Y⁹ and Y¹⁰ may be bonded together to form a ring together with the carbon atoms bonded thereto, provided that at least one of Y⁶ to Y¹⁰ represents -Z^{1b}-OH. Z^{1b} is as described above.

CF₂=CFX²-(O-R^{1d})ₙ₋₁-F Formula p2

In this formula, n, R^{1d} and X² are as described above.

When the compound p1 is reacted with the compound p2, the -Z^{1b}-OH of the compound p1 is converted to the group f1-1.

Commercially available compounds may be used as the compound p1 and the compound p2.

Examples of the method used for reacting the compound p1 and the compound p2 include methods in which the compound p1 and the compound p2 are mixed and stirred with a solvent and potassium carbonate. The reaction temperature is, for example, within a range from 20 to 80°C. The reaction time is, for example, within a range from 1 to 24 hours.

Following reaction, purification and removal of the liquid solvent and the like may be conducted as necessary.

### [Conductive Film]

The conductive film of the present disclosure has a first region, and a second region that exhibits a higher light transmittance than that of the first region. Further, the conductive film of the present disclosure has a first film containing a conductive material, and a second film containing the compound of the present disclosure, wherein the first film is disposed so as to overlap at least the first region from among the first region and the second region, and the second film is disposed so as to overlap the second region.

The conductive film according to an embodiment of the present invention and a method for producing the conductive film are described below with reference to FIGS. 1 to 8.

FIGS. 1 and 2 are explanatory diagrams relating to a conductive film 1 of an embodiment of the present invention. FIG. 1 is a plan view of the conductive film 1, and FIG. 2 is a cross-sectional view viewed in the direction of the arrows of the line II-II of FIG. 1.

As illustrated in FIGS. 1 and 2, the conductive film 1 has a first film 10, a second film 15, and a third film 20.

The conductive film 1 is provided on a substrate 50. The substrate 50 acts as the target on which the conductive film 1 is formed. The substrate 50 exhibits light transmittance.

The third film 20 is provided on top of the substrate 50. The first film 10 is provided on top of the third film 20. The second film 15 is provided between the first film 10 and the third film 20.

The conductive film 1 has a first region 10A, and a second region 10B that exhibits a higher light transmittance than that of the first region 10A.

In the conductive film 1 of this embodiment, the first film 10 is positioned in the first region 10A. The first film 10 is not present in the second region 10B.

The lower surface 11a of the first film 10 contacts the third film 20. Further, side surfaces 11b of the first film 10 contact the second film 15.

As illustrated in FIG. 1, in the conductive film 1, the plurality of second regions 10B are arranged in a matrix-like pattern, with a lattice-like first region 10A, namely a lattice-like first film 10 located in the remaining portions. In FIG. 1, the second regions 10B are illustrated as rectangular when viewed in plan view, but the invention is not limited to this configuration, and various plan view shapes may be employed depending on the design.

### (First Film)

The first film 10 is a film containing a conductive material.

There are no particular limitations on the conductive material of the first film 10, provided the material exhibits conductivity, but the material preferably contains silver (Ag), gold (Au), copper (Cu), aluminum (Al), magnesium (Mg), zinc (Zn), indium (In), tin (Sn), or ytterbium (Yb) or the like. Specific examples include metals such as Ag, Au, Cu, Al, Mg, magnesium-silver (MgAg) and ytterbium-silver (YbAg), and conductive metal oxides such as indium-tin oxide (ITO), indium-zinc oxide (IZO) and zinc oxide (ZnO). Because the compound of the present disclosure exhibits superior Ag repellency, the conductive material of the first film 10 preferably contains Ag.

The first film 10 is preferably formed with sufficient thickness to ensure the conductivity of the entire conductive film 1. The first film 10 may be any film capable of ensuring the required conductivity, and may exhibit light transmittance or lack light transmittance.

The thickness of the first film 10 is, for example, within a range from 10 to 1,000 nm. A thin first film 10 formed with a thickness of 50 nm or less exhibits light transmittance.

The first film 10 may also be composed of a thick-film section located in the first region 10A (corresponding with the first film 10 in FIGS. 1 and 2), and a thin-film section located in the second region 10B. The lower surface of this thin-film section contacts the second film 15.

The thin-film section is thinner than the thick-film section, and is formed with a thickness that ensures light transmittance.

The thickness of the thin-film section is, for example, within a range from 0 to 50 nm.

In terms of achieving superior transparency in the second region 10B of the conductive film 1, there is preferably no thin-film section.

### (Second Film)

The second film 15 is a film containing the compound of the present disclosure.

The second film 15 is disposed so as to overlap with the second region 10B. As described in the method for producing a conductive film described below, the second film 15 is used to enable formation of the first film 10 by deposition.

The compound of the present disclosure transmits light in the visible light wavelength region and repels conductive materials such as metals during deposition, meaning it is difficult for conductive materials to form a film on the compound. Accordingly, even if the second film 15 overlaps with the second region 10B that exhibits light transmittance, the film does not inhibit the light transmittance of the second region 10B.

The second film 15 is typically formed with a thickness that is equal to or thinner than the maximum thickness of the first film 10.

The thickness of the second film 15 is preferably within a range from 10 to 500 nm, and more preferably from 10 to 100 nm. Provided the thickness of the second film 15 is at least as large as the above lower limit, the film exhibits particularly superior repellency of conductive materials such as metals, whereas provided the thickness is not more than the above upper limit, the light transmittance of light within the visible light wavelength region is particularly superior.

### (Third Film)

In this embodiment 1, the third film 20 is provided on a surface 50a of the substrate 50. The third film 20 is a film that contains a conductive material.

Examples of the conductive material of the third film 20 include the same materials as those exemplified above for the conductive material of the first film 10. The conductive material of the first film 10 and the conductive material of the third film 20 may be the same or different.

The third film 20 has light transmittance. The third film 20 exhibits higher light transmittance than the first film 10. The thickness of the third film 20 is, for example, within a range from 0 to 50 nm.

In those cases where the first film 10 has a thin-film section, the third film 20 overlaps with the thin-film section when viewed in plan view. Accordingly, the combined thickness of the third film 20 and the thin-film section should be adjusted appropriately to ensure that the second region 10B has light transmittance.

The third film 20 can also be used alone as a transparent electrode used in various photoelectronic devices such as light-emitting devices and light-receiving devices. However, a third film 20 that has been formed thin enough to ensure light transmittance has high wiring resistance.

In contrast, in the conductive film 1 of this embodiment of the present invention, the third film 20 and the first film 10 are layered. Accordingly, in the location that overlaps with the first region 10A, the first film 10 and the third film 20 are layered and form a thicker film. As a result, the resistance of the overall conductive film 1 can be reduced while ensuring light transmittance in the location that overlaps with the second region 10B.

### (Method for Producing Conductive Film)

FIGS. 3 to 5 are schematic diagrams illustrating a method for producing the conductive film 1 described above.

First, as illustrated in FIG. 3, a conductive material is formed on the surface of the substrate 50, thus obtaining the third film 20 (step a).

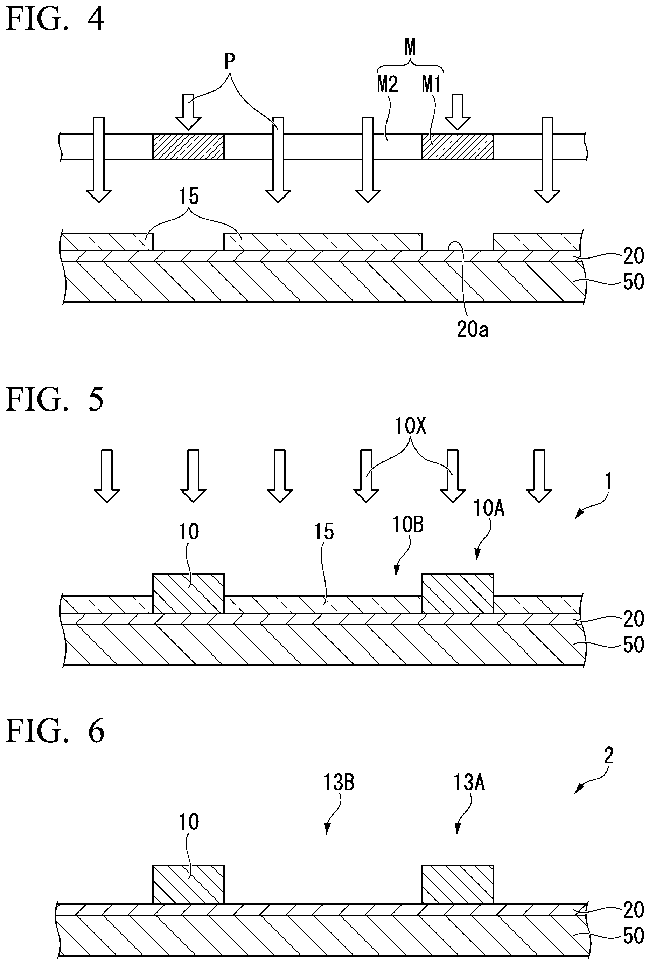

Next, as illustrated in FIG. 4, a compound P of the present disclosure is deposited via a mask M onto the surface 20a of the third film 20 on the substrate 50, thus forming the second film 15 (step b).

Subsequently, as illustrated in FIG. 5, a conductive material 10X is deposited from above the second film 15, thus forming the first film 10 (step c).

### <Step a>

The third film 20 can be formed using a dry coating method such as vapor deposition, sputtering, CVD (chemical vapor deposition), or ALD (atomic layer deposition) or the like.

### <Step b>

The mask M has open sections M2 arranged in a matrix pattern, and a shielded section M1 provided in a lattice shape.

Deposition of the compound P is conducted by heating the compound P under reduced pressure, for example at a pressure of 1×10⁻³ Pa or lower. The heating temperature is, for example, within a range from 150 to 300°C.

By depositing the compound P via the mask M, the compound P passes through the open sections M2 of the mask M and reaches the surface 20a of the third film 20, but is blocked by the shielded section M1 of the mask M. As a result, the second film 15 containing the compound P of the present disclosure is formed.

The second film 15 is arranged on the surface 20a in a matrix-like pattern. Further, the third film 20 is exposed in a lattice-like shape in those locations in which the second film 15 has not been formed.

### <Step c>

The conductive material 10X is deposited from above the second film 15.

The conductive material 10X is unlikely to adhere to the surface of the second film 15 containing the compound of the present disclosure, but adheres readily to the surface of the third film 20 (or the substrate 50). Accordingly, by depositing the conductive material 10X across the entire surface from above the substrate 50 without using a mask, the conductive material 10X is formed as a comparatively thick film on the surface of the third film 20, and formed as a comparatively thin film, or not formed at all, on the surface of the second film 15. This leads to formation of the first film 10.

This completes production of the conductive film 1.

By producing a conductive film with the type of configuration described above, a novel conductive film having two types of regions with different light transmittances is obtained.

Further, by using the type of conductive film production method described above, this type of conductive film can be produced with ease.

In this embodiment, the second film 15 is present in the second region 10B of the conductive film 1, but the method may also have a step for removing the second film 15 after the step c illustrated in FIG. 5. In those cases where a thin-film section of the first film 10 is present in the second region 10B, removal of the second film 15 enables the second film 15 and the thin-film section to be removed simultaneously.

FIG. 6 is a schematic diagram illustrating a conductive film 2 obtained following removal of the second film 15, and corresponds with FIG. 2. As illustrated in FIG. 6, the conductive film 2 has a first film 13 and the third film 20. In a first region 13A of the conductive film 2, the lattice-shaped first film 10 and the third film 20 overlap. Further, in a second region 13B of the conductive film 2, only the third film 20 is present.

In a conductive film 2 having this type of configuration, the light transmittance of the second region 13B is higher than even the light transmittance of the second region 10B of the conductive film 1 described above, increasing the contrast between the first region 13A and the second region 13B.

Examples of the method used for removing the second film 15 include methods of dissolving or stripping the second film 15 using a solvent, and methods of removing the second film 15 by dry etching.

The solvent used is preferably a solvent that dissolves the second film 15 but essentially has no dissolution effect on the other materials constituting the substrate on which the conductive film 1 is formed. This enables the compound to be removed without impairing the quality of the photoelectronic device.

Examples of solvents that may be used to remove the second film 15 include acetone, methyl ethyl ketone, chloroform, dichloromethane, and carbon tetrachloride.

Furthermore, in this embodiment, the third film 20 was included, but the third film 20 could also be excluded.

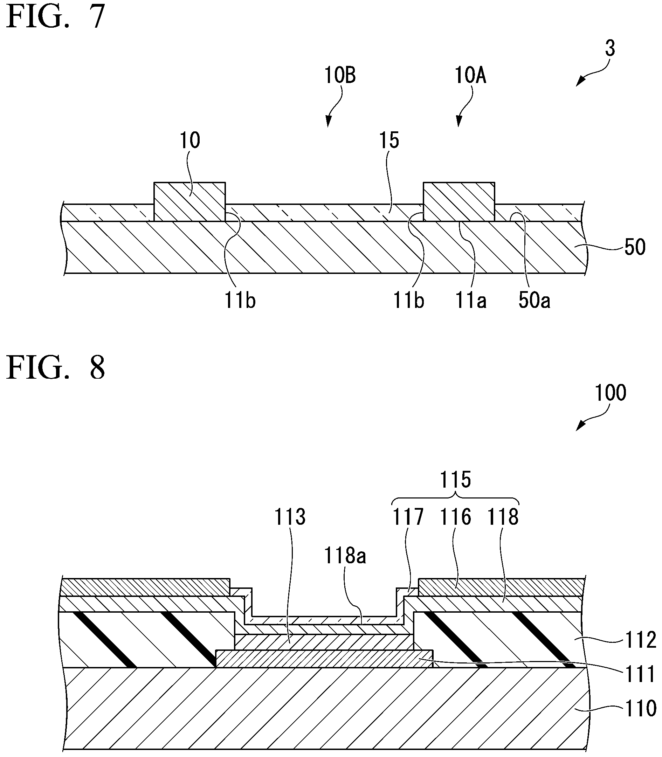

FIG. 7 is an explanatory diagram illustrating a modification of the embodiment of the present invention, and corresponds with FIG. 2.

The conductive film 3 illustrated in FIG. 7 has the first film 10 and the second film 15. The first film 10 is provided on the substrate 50. The second film 15 is provided between the first film 10 and the substrate 50.

The lower surface 11a of the first film 10 contacts the substrate 50. Further, the side surfaces 11b of the first film 10 contact the second film 15.

This type of conductive film 3 can be produced in a similar manner to the conductive film production method described above, with the exception of excluding the step illustrated in FIG. 3 (step a), and depositing the compound P of the present disclosure directly onto the surface 50a of the substrate 50.

Moreover, following production of the conductive film 3 via the step illustrated in FIG. 5 (step c), the above step of removing the second film 15 may also be included.

### [Photoelectronic Device]

The photoelectronic device of the present disclosure has the conductive film of the present disclosure.

This photoelectronic device is preferably an organic EL device containing a substrate, an anode provided on the substrate, a cathode provided on the substrate, and an active layer disposed between the anode and the cathode, wherein the cathode is the conductive film of the present disclosure.

FIG. 8 is a cross-sectional schematic view illustrating an organic EL device 100 (a photoelectronic device according to an embodiment of the present invention). The organic EL device 100 has a substrate 110, an anode 111, a partition wall 112, a functional layer 113, and a cathode 115 in a stacked structure. The functional layer 113 includes a light-emitting layer.

The organic EL device 100 of this embodiment employes a top emission system in which light generated in the functional layer 113 is emitted externally through the cathode 115.

### (Substrate)

The substrate 110 may have light transmittance, or may lack light transmittance. Examples of materials that may be used in forming the substrate 110 include inorganic materials such as glass, quartz glass and silicon nitride, and organic polymers (resins) such as polyimide resins, acrylic resins and polycarbonate resins. Further, provided the insulating properties of the surface can be ensured, a metal material may also be used for forming the substrate 110.

Furthermore, the substrate 110 is also provided with various wiring and driver elements (not shown in the figure) that are connected electrically to the organic EL device.

### (Anode)

The anode 111 is formed on top of the substrate 110, and supplies holes to the functional layer 113. Further, the anode 111 has a light reflecting property which reflects the light emitted from the light-emitting layer included in the functional layer 113.

The anode 111 contains a conductive material.

Examples of materials that can be used as the conductive material include conductive metal oxides such as ITO and IZO. Furthermore, in order to impart the anode 111 with a light reflecting property, a reflective film containing a metal may be provided on the substrate 110 side of the anode 111 or the functional layer 113 side of the anode 111. In other words, the anode 111 may have a layered structure that includes a layer containing a conductive metal oxide and a reflective film containing a metal.

Metals such as silver may also be used as the material for forming the anode 111.

The thickness of the anode 111 is not particularly limited, but is preferably within a range from 30 to 300 nm. The thickness of the anode 111 may be, for example, 100 nm.

### (Partition Wall)

The partition wall 112 overlaps the periphery of the anode 111 and is formed, for example, in a lattice shape. The functional layer 113 is formed in the opening of the partition wall 112, partitioning the organic EL element 100.

The partition wall 112 is formed, for example, from a resin such as a polyimide.

### (Functional Layer)

The functional layer 113 is formed overlapping the anode 111. The functional layer 113 has a light-emitting layer. In the light-emitting layer, holes injected from the anode 111 and electrons injected from the cathode 115 recombine and emit photons, causing light emission. The emission wavelength at that time is determined according to the material that forms the light-emitting layer. The light-emitting layer corresponds with the "active layer" in the present disclosure.

The light-emitting layer can be formed using the types of conventional materials used as the material for the light-emitting layers of organic EL devices.

A single light-emitting layer material may be used alone, or a combination of two or more such materials may be used, with the material(s) selected appropriately in accordance with the desired emission wavelength.

The functional layer 113 may have a hole injection layer and a hole transport layer between the light-emitting layer and the anode 111.

The hole injection layer has the function of facilitating injection of holes from the anode into the hole transport layer.

The hole transport layer has the function of favorably transporting the holes injected from the anode 111 toward the light-emitting layer.

The functional layer 113 may have an electron transport layer and an electron injection layer between the light-emitting layer and the cathode 115.

The electron transport layer has the function of favorably transporting the electrons injected from the cathode 115 toward the light-emitting layer.

The electron injection layer has the function of facilitating injection of electrons from the cathode 115 into the electron transport layer.

### (Cathode)

The cathode 115 is formed across the entire surface, covering the partition wall 112 and the functional layer 113. The cathode 115 has the function of injecting electrons into the functional layer.

In the photoelectronic device of this embodiment, the cathode 115 employs the conductive film described above. The cathode 115 has a first film 116, a second film 117, and a third film 118.

The third film 118 is formed covering the partition wall 112 and the functional layer 113. The structure and material for the third film 118 can employ the same structure and material as that described above for the third film 20 of the embodiment described above.

The second film 117 overlaps the functional layer 113 when viewed in plan view, and is formed on the upper surface 118a of the third film 118. The structure and material for the second film 117 can employ the same structure and material as that described above for the second film 15 of the first embodiment.

The first film 116 is formed overlapping the partition wall 112 when viewed in plan view. The structure and material for the first film 116 can employ the same structure and material as that described above for the first film 10 of the first embodiment.

In other words, the first region of the cathode 115 overlaps the partition wall 112, and the second region of the cathode 115 overlaps the functional layer 113 containing the light-emitting layer.

### (Microcavity Structure)

In the organic EL device 100 of this embodiment of the present invention, the anode 111 and the cathode 115 form an optical resonator structure (microcavity) that resonates light between the anode 111 and the cathode 115, namely between the upper surface of the reflective film of the anode 111 and the lower surface of the third film 118. The light generated in the light-emitting layer undergoes repeated reflection between the anode 111 and the cathode 115, and light with a wavelength that matches the optical path length between the anode 111 and the cathode 115 resonates and is amplified. On the other hand, light of wavelengths that do not match the optical path length between the anode 111 and the cathode 115 is attenuated.

Here, the "optical path length" is calculated using the desired light wavelength for emission from the device and the refractive index of each layer at that desired light wavelength.

The optical path length between the anode 111 and the cathode 115 is set, for example, to an integer multiple of the central wavelength of the light L generated in the light-emitting layer contained in the functional layer 113. In this case, the light L emitted from the light-emitting layer is amplified as it approaches the central wavelength and attenuated as it moves away from the central wavelength before being emitted outside the organic EL device 100. In this manner, the light L emitted from the organic EL device 100 has an emission spectrum with a narrow half-width and improved color purity.

Because the organic EL device 100 with this type of structure has a conductive film described in the present disclosure as the cathode 115, the wiring resistance of the cathode 115 can be reduced while maintaining high light extraction efficiency in the second region, thereby achieving good driving performance.

In the embodiment described above, an organic EL device was described as the photoelectronic device, but the photoelectronic device using the conductive film of the present disclosure is not limited to organic EL devices.

The photoelectronic device of the present disclosure may be, for example, a semiconductor laser. Conventional semiconductor laser structures may be used. By employing the conductive film described above for the cathode of the semiconductor laser, the resistance of the cathode can be reduced, and a semiconductor laser of improved output can be obtained.

The photoelectronic device of the present disclosure may be, for example, a light-receiving device such as a light sensor or solar cell. A conventional structure can be employed for the light sensor or solar cell, in which the active layer (the light-receiving layer) transmits holes and electrons generated in the active layer to the cathode and the anode via semiconductor layers in accordance with the intensity of the light it receives.

By employing the conductive film described above as the electrodes (anode and cathode) of the light sensor or solar cell, a light sensor with improved detection performance or a solar cell having improved power generation efficiency can be obtained.

The conductive film and photoelectronic device of the present disclosure provide improved transparency for the overall device, and are therefore useful in applications such as transparent displays. Furthermore, in under-display cameras and under-display sensors and the like, because it is necessary to increase the light transmittance of the display above the camera or sensor, the conductive film of the present disclosure is extremely useful.

In addition to use in photoelectronic devices, the conductive film of the present disclosure can also be used in patterned electrodes and wiring.

Although preferred embodiments according to the present disclosure have been described above, the present disclosure is not limited to these examples. The shapes and combinations of the various constituent components shown in the above examples are merely examples, and can be modified in various ways based on design requirements and the like, without departing from the scope of the present disclosure.

The preferred aspects of the present disclosure described above include the following embodiments.
[1A] A compound having a molecular weight within a range from 500 to 5,000,
   composed of hydrogen atoms, carbon atoms, oxygen atoms and fluorine atoms, and
   having at least one alicyclic structure, at least one aromatic ring structure bonded directly to the alicyclic structure, and at least one monovalent fluorine-containing aliphatic group bonded directly to the aromatic ring structure.
[2A] The compound according to [1A], wherein the number of the alicyclic structures is two or more.
[3A] The compound according to [1A] or [2A], wherein the alicyclic structure is monocyclic.
[4A] The compound according to any one of [1A] to [3A], wherein the monovalent fluorine-containing aliphatic group is represented by formula fl shown below:

   -Z¹-(O-R¹)ₙ-F Formula fl

   wherein R¹ represents an alkylene group or a fluoroalkylene group, n represents an integer of 2 or greater, the R¹ groups may be the same or different, and Z¹ represents a single bond or a divalent linking group that does not contain an aromatic ring structure.
[5A] The compound according to any one of [1A] to [4A], represented by formula 1 shown below: 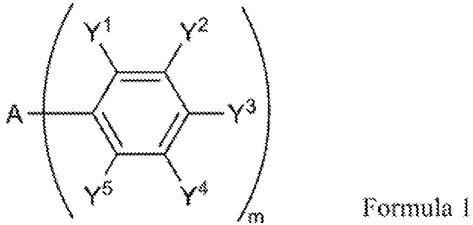 wherein m represents an integer within a range from 1 to 8, A represents an m-valent group containing the at least one alicyclic structure, Y¹ to Y⁵ each independently represent the monovalent fluorine-containing aliphatic group, a hydrogen atom, a fluorine atom, or a monovalent aliphatic group having no fluorine atom, and one or two of the combinations of Y¹ and Y², Y² and Y³, Y³ and Y⁴, and Y⁴ and Y⁵ may be bonded together to form a ring together with the carbon atoms bonded thereto, provided that at least one of Y ¹ to Y⁵ represents the monovalent fluorine-containing aliphatic group.
[6A] The compound according to [5A], wherein
   m in the formula 1 represents 2, and A is a group represented by formula A-1a shown below, or
   m in the formula 1 represents 3, and A represents a group represented by formula A-2a shown below, or
   m in the formula 1 represents 4, and A represents a group represented by formula A-3a shown below: 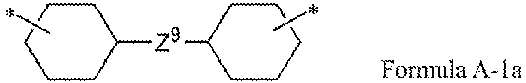 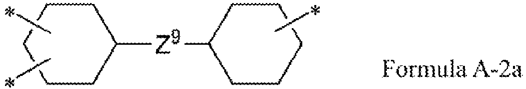 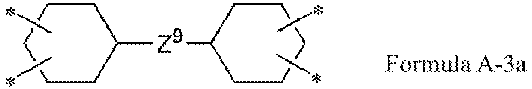 wherein Z⁹ represents a single bond or a divalent linking group that does not contain an aromatic ring structure, and * represents a bonding site.
[7A] A conductive film having a first region, and a second region that exhibits a higher light transmittance than that of the first region, wherein
   the conductive film has a first film containing a conductive material, and a second film containing the compound according to any one of [1A] to [6A], and
   the first film is disposed so as to overlap at least the first region from among the first region and the second region, and the second film is disposed so as to overlap the second region.
[8A] The conductive film according to [7A], wherein the conductive material contains silver.
[9A] A photoelectronic device having the conductive film according to [7A] or [8A].
[10A] The photoelectronic device according to [9], having
   a substrate,
   an anode provided on the substrate,
   a cathode provided on the substrate, and
   an active layer disposed between the anode and the cathode, wherein
   the cathode is the conductive film.
[11A] A method for producing a conductive film, the method including:
   depositing the compound according to any one of [1A] to [6A] on a substrate via a mask to form a second film containing the compound, and
   depositing a conductive material from above of the second film.
[12A] The method for producing a conductive film according to [11A], wherein the conductive material contains silver.

### [Examples]

The present disclosure is described below in further detail using a series of examples and comparative examples, but the present disclosure is not limited to the following examples, provided it does not exceed the scope of the disclosure. Examples 1 to 20 are examples according to the present disclosure, and Examples 21 to 25 are comparative examples.

### [Synthesis of Compounds]

### (Example 1)

A compound 1-1 was produced using the procedure described below.

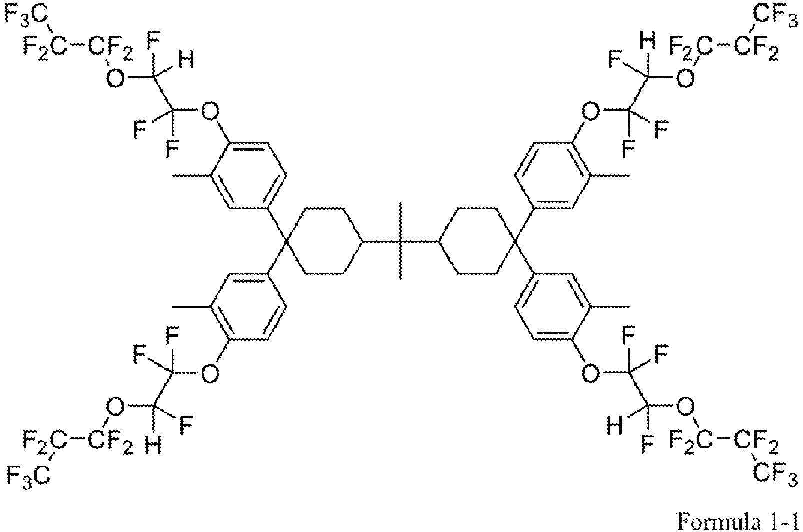

First, 1.03 g (1.63 mmol) of 4,4',4",4‴-[(1-methylethylidene)di-4-cyclohexanyl-1-ylidene]tetrakis[2-ethylphenol] (manufactured by Honshu Chemical Industry Co., Ltd., hereinafter also abbreviated as "tekOC-4HBPA") was weighed into a 50 mL two-neck round-bottom flask and dissolved in a mixed solvent containing 7 mL of N,N-dimethylformamide (hereinafter also abbreviated as "DMF") and 2 mL of 2-propanol. Subsequently, 1.1 g (8 mmol) of potassium carbonate was added and stirred to form a suspension. Next, 2.7 mL (16 mmol) of perfluoropropoxyethylene was added, and the resulting mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 2.15 g of the compound 1-1 in the form of a colorless glass-like solid (yield: 78%). The identification data for the compound 1-1 are shown below.
¹H-NMR (acetone-D6): δ 7.5 to 6.9 (m, 16H), 2.8 (m, 4H), 2.3 (s, 6H), 2.2 (s, 6H), 1.9 (m, 4H), 1.7 (m, 4H), 1.5 (m, 2H), 1.2 (m, 4H), 0.5 (s, 6H)
¹⁹F-NMR (acetone-D6): δ -82 (s, 12F), -86 (m, 16F), -131 (s, 8F), -146 (m, 4F)

### (Example 2)

A compound 1-2 was produced using the procedure described below.

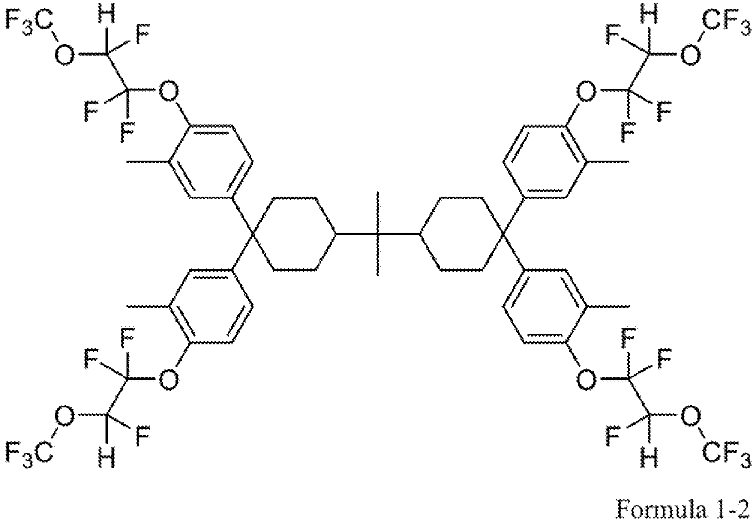

First, 1.05 g (1.66 mmol) of tekOC-4HBPA was weighed into a 50 mL two-neck round-bottom flask and dissolved in a mixed solvent containing 7 mL of DMF and 2 mL of 2-propanol. Subsequently, 1.1 g (8 mmol) of potassium carbonate was added and stirred to form a suspension. The flask openings were sealed with septa, perfluoromethyl vinyl ether that had been transferred into a 3 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 1.51 g of the compound 1-2 in the form of a colorless glass-like solid (yield: 70%). The identification data for the compound 1-2 are shown below.
¹H-NMR (acetone-D6): δ 7.5 to 7.1 (m, 12H), 6.9 to 6.7 (m, 4H), 2.8 (m, 4H), 2.3 (s, 6H), 2.2 (s, 6H), 1.9 (m, 4H), 1.7 (m, 4H), 1.5 (m, 2H), 1.2 (m, 4H), 0.5 (s, 6H)
¹⁹F-NMR (acetone-D6): δ -60 (s, 12F), -86 (m, 8F), -146 (m, 4F)

### (Example 3)

A compound 1-3 was produced using the procedure described below.

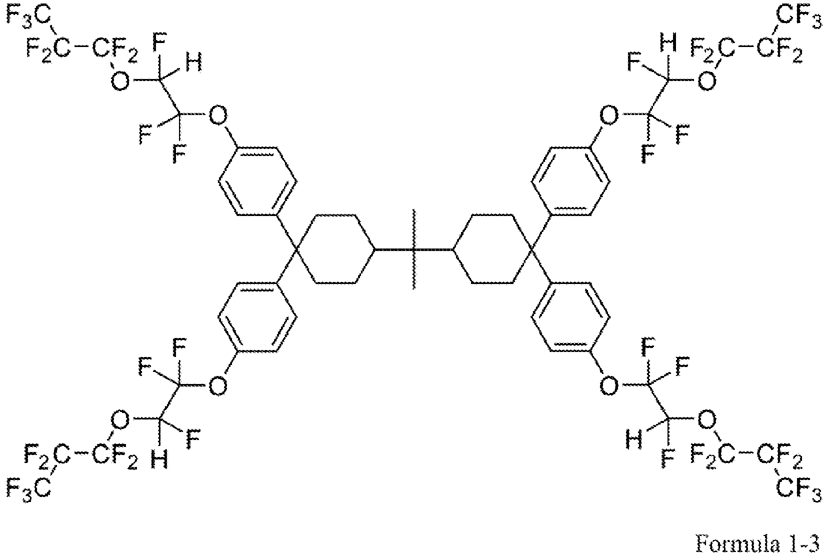

First, 2.00 g (3.5 mmol) of 4,4',4",4‴-[(1-methylethylidene)di-4-cyclohexanyl-1-ylidene]tetrakisphenol (manufactured by Honshu Chemical Industry Co., Ltd., hereinafter also abbreviated as "tekP-4HBPA") was weighed into a 100 mL two-neck round-bottom flask and dissolved in a mixed solvent containing 14 mL of DMF and 4 mL of 2-propanol. Subsequently, 2.4 g (17 mmol) of potassium carbonate was added and stirred to form a suspension. Next, 7.1 mL (42 mmol) of perfluoropropoxyethylene was added, and the resulting mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 4.27 g of the compound 1-3 in the form of a colorless glass-like solid (yield: 75%). The identification data for the compound 1-3 are shown below.
¹H-NMR (acetone-D6): δ 7.6 (m, 4H), 7.4 (m, 4H), 7.3 (m, 4H), 7.2 (m, 4H), 7.0 to 6.8 (m, 4H), 2.8 (m, 4H), 1.9 (m, 4H), 1.7 (m, 4H), 1.5 (m, 2H), 1.2 (m, 4H), 0.5 (s, 6H)
¹⁹F-NMR (acetone-D6): δ -82 (s, 12F), -86 (m, 16F), -130 (s, 8F), -146 (m, 4F)

### (Example 4)

A compound 1-4 was produced using the procedure described below.

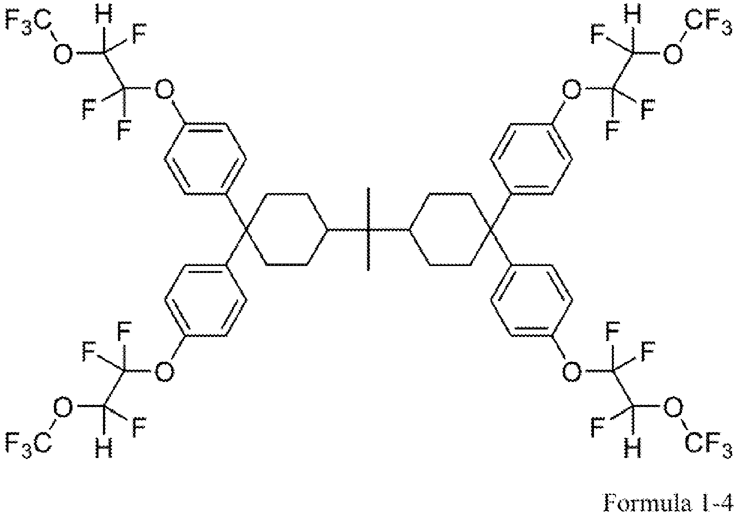

First, 2.00 g (3.5 mmol) of tekP-4HBPA was weighed into a 100 mL two-neck round-bottom flask and dissolved in a mixed solvent containing 14 mL of DMF and 3.5 mL of 2-propanol. Subsequently, 2.4 g (17 mmol) of potassium carbonate was added and stirred to form a suspension. The flask openings were sealed with septa, perfluoromethyl vinyl ether that had been transferred into a 5 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 2.80 g of the compound 1-4 in the form of a colorless glass-like solid (yield: 65%). The identification data for the compound 1-4 are shown below.
¹H-NMR (acetone-D6): δ 7.6 (m, 4H), 7.4 (m, 4H), 7.3 (m, 4H), 7.2 (m, 4H), 6.8 to 6.6 (m, 4H), 2.8 (m, 4H), 1.9 (m, 4H), 1.7 (m, 4H), 1.5 (m, 2H), 1.2 (m, 4H), 0.5 (s, 6H)
¹⁹F-NMR (acetone-D6): δ -60 (s, 12F), -86 (m, 8F), -146 (m, 4F)

### (Example 5)

A compound 1-5 was produced using the procedure described below.

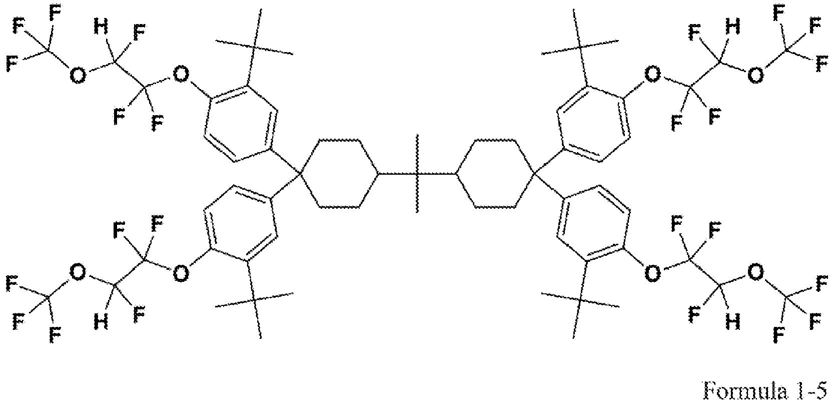

First, 2.80 g (3.5 mmol) of 4,4',4",4‴-(propane-2,2-diylbis(cyclohexane-4,1,1-triyl))tetrakis(2-(ter-butyl)phenol) (manufactured by Honshu Chemical Industry Co., Ltd., hereinafter also abbreviated as "tekOTBP-4HBPA") was weighed into a 100 mL two-neck round-bottom flask and dissolved in a mixed solvent containing 14 mL of DMF and 3.5 mL of 2-propanol. Subsequently, 2.4 g (17 mmol) of potassium carbonate was added and stirred to form a suspension. The flask openings were sealed with septa, perfluoromethyl vinyl ether that had been transferred into a 5 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 3.08 g of the compound 1-5 in the form of a colorless glass-like solid (yield: 60%). The identification data for the compound 1-5 are shown below.
¹H-NMR (CDCl₃): δ 7.5 to 6.9 (m, 12H), 6.1 to 5.8 (m, 4H), 2.6 (m, 4H), 1.9 (m, 4H), 1.6 (m, 4H), 1.5 to 1.1 (m, 42H), 0.5 (s, 6H)
¹⁹F-NMR (CDCl₃): δ -57 (s, 12F), -82 (m, 8F), -142 (m, 4F)

### (Example 6)

A compound 1-6 was produced using the procedure described below.

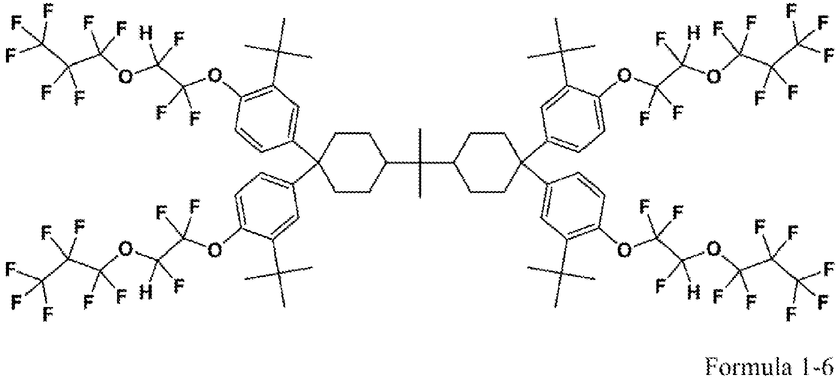

First, 2.80 g (3.5 mmol) of tekOTBP-4HBPA was weighed into a 100 mL two-neck round-bottom flask and dissolved in a mixed solvent containing 14 mL of DMF and 4 mL of 2-propanol. Subsequently, 2.4 g (17 mmol) of potassium carbonate was added and stirred to form a suspension. Next, 7.1 mL (42 mmol) of perfluoropropoxyethylene was added, and the resulting mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 3.59 g of the compound 1-6 in the form of a colorless glass-like solid (yield: 70%). The identification data for the compound 1-6 are shown below.
¹H-NMR (CDCl₃): δ 7.5 to 6.9 (m, 12H), 6.1 to 5.8 (m, 4H), 2.6 (m, 4H), 1.9 (m, 4H), 1.6 (m, 4H), 1.5 to 1.1 (m, 42H), 0.5 (s, 6H)
¹⁹F-NMR (CDCl₃): δ -79 (m, 12F), -81 to -85 (m, 16F), -127 (s, 8F), -140 (m, 4F)

### (Example 7)

A compound 1-7 was produced using the procedure described below.

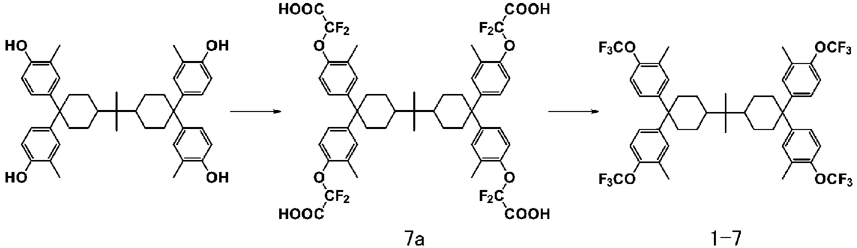

A two-neck flask that had been flushed with nitrogen was charged with 2.5 g (63 mmol) of sodium hydride (60% by mass in oil) and 40 mL of dioxane, and a dioxane solution containing 5.0 g (7.9 mmol) of tekOC-4HBPA was then added gradually to the flask. Subsequently, the reaction mixture was cooled to 0°C, 6.2 g (47 mmol) of chlorodifluoroacetic acid was added gradually, and the resulting mixture was then heated to 100°C and stirred under heat for 24 hours. The reaction liquid was then cooled by standing at 25°C, and was then added dropwise to an aqueous solution of saturated ammonium chloride to halt the reaction. Hydrochloric acid was added to adjust the pH to 1, and sodium hydrogen carbonate was then added to adjust the pH to 8. Following washing with dichloromethane, hydrochloric acid was added to adjust the pH to 1, and the product was extracted with dichloromethane. The solution was dried over magnesium sulfate and then concentrated under reduced pressure, yielding 8.5 g of a crude product of a compound 7a. This crude product was used, as is, in the subsequent step.

A two-neck flask that had been flushed with nitrogen was charged with 22.4 g (63 mmol) of Selectfluor, 1.6 g (6.3 mmol) of silver trifluoromethanesulfonate, 8.5 g of the crude product of the compound 7a, 36 mL of dichloromethane, 4 mL of water, and 14 g (126 mmol) of trifluoroacetic acid, and the resulting mixture was stirred under heat at 55°C for 8 hours. Following completion of the reaction, the reaction mixture was cooled by standing at 25°C, water was added, and a separation operation was performed. The thus obtained organic layer was washed sequentially with water and a saturated brine solution. Following drying over magnesium sulfate, the solvent was removed by distillation under reduced pressure, and the product was purified by silica gel column chromatography (developing solvent: hexane/ethyl acetate = 95/5), yielding 1.1 g of the compound 1-7 (yield: 15%). The identification data for the compound 1-7 are shown below.
¹H-NMR (CDCl₃): δ 7.2 to 6.8 (m, 12H), 2.7 to 2.5 (m, 4H), 2.2 (s, 6H), 2.1 (s, 6H), 1.9 to 1.7 (m, 4H), 1.6 to 1.5 (m, 4H), 1.4 to 1.3 (m, 2H), 1.3 to 1.1 (m, 4H), 0.5 (s, 6H)
¹⁹F-NMR (CDCl₃): δ -41 (s, 6F), -42 (s, 6F)

### (Example 8)

A compound 1-8 was produced using the procedure described below.

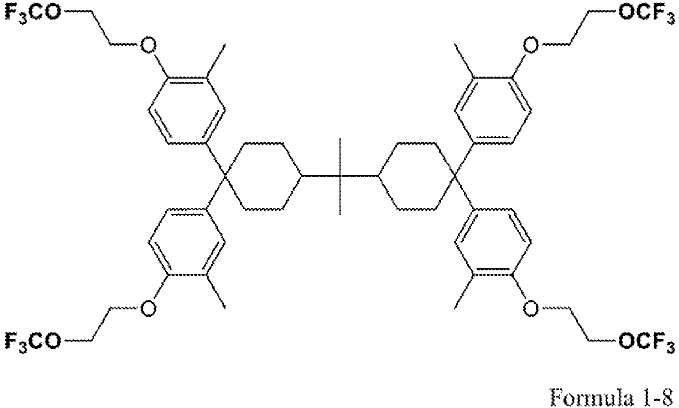

First, 1.0 g (1.6 mmol) of tekOC-4HBPA was weighed into a 50 mL two-neck round-bottom flask and dissolved in 8 mL of DMF. Subsequently, 6.3 g (19 mmol) of cesium carbonate and 3.7 g (19 mmol) of 1-bromo-3-(trifluoromethoxy)propane were added, and the resulting mixture was stirred at 70°C for 12 hours. Following a separation operation using water/ethyl acetate, the product was purified by silica gel column chromatography (developing solvent: hexane/ethyl acetate = 95/5), and the solvent was then removed under reduced pressure, yielding 0.61 g of the compound 1-8 (yield: 35%). The identification data for the compound 1-8 are shown below.
¹H-NMR (CDCl₃): δ 7.2 to 6.4 (m, 12H), 4.4 to 4.1 (m, 8H), 4.0 to 3.7 (m, 8H), 2.7 to 2.5 (m, 4H), 2.2 (s, 6H), 2.1 (s, 6H), 1.9 to 1.7 (m, 4H), 1.6 to 1.5 (m, 4H), 1.4 to 1.3 (m, 2H), 1.3 to 1.1 (m, 4H), 0.5 (s, 6H)
¹⁹F-NMR (CDCl₃): δ -59 (s, 6F), -60 (s, 6F)

### (Example 9)

A compound 1-9 was produced using the procedure described below.

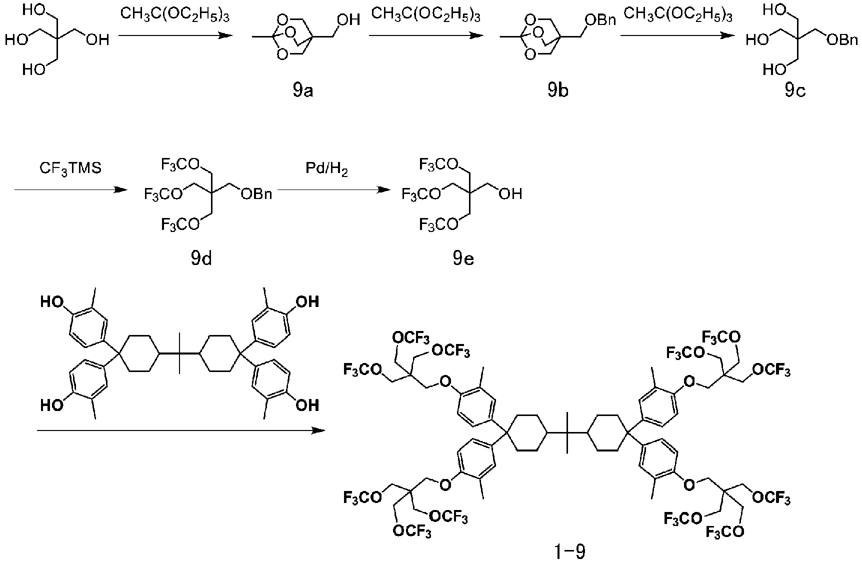

### <Synthesis of Compounds 9a, 9b, 9c>

Compounds 9c, 9d and 9e were synthesized using the method disclosed in Tetrahedron 2007, 63, 3982 to 3988.

### <Synthesis of Compound 9d>

In a glove box under a nitrogen atmosphere, a 300 mL round-bottom flask was charged with 1.0 g (4.4 mmol) of the compound 9c, 17 g (66 mmol) of silver trifluoromethanesulfonate, 9.4 g (27 mmol) of Selectfluor (a registered trademark), 12 g (80 mmol) of cesium fluoride, 8.4 g (27 mmol) of N-fluorobenzenesulfonimide, and 90 g (972 mmol) of toluene, and the resulting mixture was stirred. To the thus obtained mixture were added 6.4 g (66 mmol) of 2-fluoropyridine and 9.4 g (66 mmol) of trifluoromethyltrimethylsilane, and the resulting mixture was stirred for 16 hours. Following completion of the reaction, the reaction liquid was passed through 30 g of silica gel and eluted with ethyl acetate. The resulting solution was concentrated under reduced pressure and purified by silica gel column chromatography (hexane : ethyl acetate = 9:1), yielding the compound 9d (1.4 g, yield: 74%). The identification data for the compound 9d are shown below.
¹H-NMR (CDCl₃): δ 7.4 to 7.2 (m, 5H), 4.5 (s, 2H), 4.1 (s, 6H), 3.5 (2, 2H)
¹⁹F-NMR (CDCl₃): δ -59 (s, 9F)

### <Synthesis of Compound 9e>

A 100 mL round-bottom flask was charged with 1.4 g (3.3 mmol) of the compound 9d and 20 mL of methanol, and 1.4 g of Pd/C (palladium-carbon with a palladium content of 5% by mass) (contains water) was then added. Subsequently, the interior of the flask was flushed three times with hydrogen, and the reaction mixture was stirred at 25°C for 16 hours. The Pd/C was then removed by celite filtration, the solvent was removed from the filtrate under reduced pressure, and the resulting crude product was purified by silica gel column chromatography (hexane : ethyl acetate = 10:1), yielding 1.0 g of the compound 9e (yield: 90%). The identification data for the compound 9e are shown below.
¹H-NMR (CDCl₃): δ 4.1 (s, 6H), 3.8 (d, 2H), 1.6 (t, 1H)
¹⁹F-NMR (CDCl₃): δ -59 (s, 9F)

### <Synthesis of Compound 1-9>

A 25 mL vial was charged with 1.0 g (1.6 mmol) of tekOC-4HBPA, 2.5 g (9.6 mmol) of triphenylphosphine, 6 mL of tetrahydrofuran (THF), 1 g of 4 Å molecular sieves and 3.3 g (9.6 mmol) of the compound 9d, and the resulting mixture was stirred at 0°C. Subsequently, 4.4 mL of diethyl azodicarboxylate (DEAD) (2.2 M toluene solution) was added, and following sealing of the vial, the reaction mixture was stirred under heat at 45°C for 24 hours. Following cooling by standing at 25°C, THF was added, and the reaction mixture was then filtered and concentrated under reduced pressure, yielding a crude product. Purification of this crude product by silica gel column chromatography (hexane) yielded 1.1 g of the compound 1-9 (yield: 35%). The identification data for the compound 1-9 are shown below.
¹H-NMR (CDCl₃): δ 7.2 to 6.4 (m, 12H), 4.2 to 4.0 (m, 8H), 3.8 to 3.5 (m, 24H), 2.7 to 2.5 (m, 4H), 2.2 (s, 6H), 2.1 (s, 6H), 1.9 to 1.7 (m, 4H), 1.6 to 1.5 (m, 4H), 1.4 to 1.3 (m, 2H), 1.3 to 1.1 (m, 4H), 0.5 (s, 6H)
¹⁹F-NMR (CDCl₃): δ -59 (s, 18F), -60 (s, 18F)

### (Example 10)

A compound 1-10 was produced using the procedure described below.

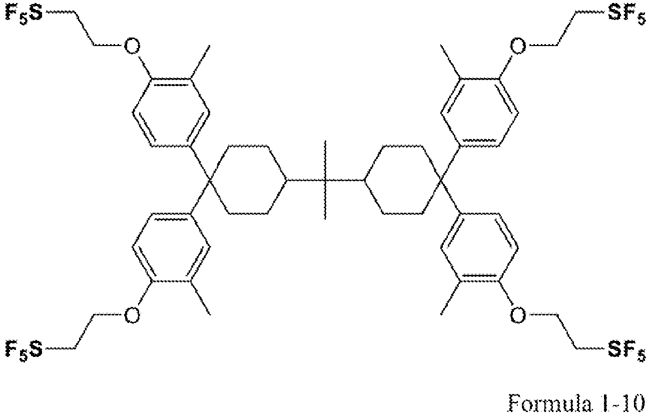

First, 1.0 g (1.6 mmol) of tekOC-4HBPA was weighed into a 50 mL two-neck round-bottom flask and dissolved in a 8 mL of DMF. Subsequently, 6.3 g (19 mmol) of cesium carbonate and 2.3 g (9.6 mmol) of 2-bromoethylsulfur pentafluoride were added, and the resulting mixture was stirred at 70°C for 12 hours. Following a separation operation using water/ethyl acetate, the product was purified by silica gel column chromatography (developing solvent: hexane/ethyl acetate = 95/5), and the solvent was then removed under reduced pressure, yielding 0.60 g of the compound 1-10 (yield: 30%). The identification data for the compound 1-10 are shown below.
¹H-NMR (CDCl₃): δ 7.2 to 6.4 (m, 12H), 4.3 to 4.0 (m, 8H), 3.7 to 3.1 (m, 8H), 2.7 to 2.5 (m, 4H), 2.2 (s, 6H), 2.1 (s, 6H), 1.9 to 1.7 (m, 4H), 1.6 to 1.5 (m, 4H), 1.4 to 1.3 (m, 2H), 1.3 to 1.1 (m, 4H), 0.5 (s, 6H)
¹⁹F-NMR (CDCl₃): δ 88 to 83 (m, 4F), 70 to 68 (m, 16F)

### (Example 11)

A compound 1-11 was produced using the procedure described below.

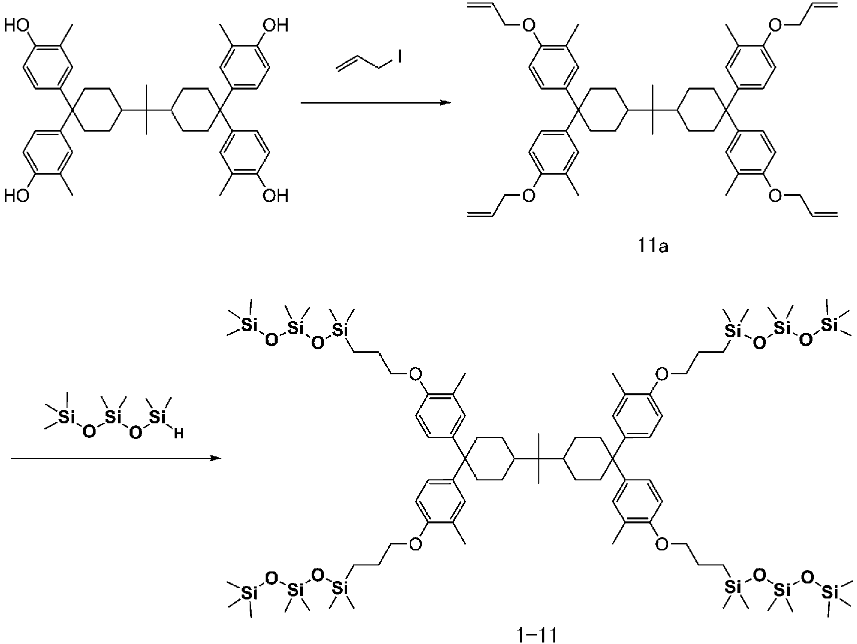

### <Synthesis of Compound 11a>

First, 10.0 g (15.8 mmol) of tekOC-4HBPA was weighed into a 500 mL two-neck round-bottom flask and dissolved in 300 mL of acetone. Subsequently, 21.6 g (157 mmol) of potassium carbonate was added and stirred to form a suspension. Next, 11.5 mL (126 mmol) of allyl iodide was added, and the reaction mixture was stirred at 80°C for 12 hours. Following separation of the solids by filtration, the filtrate was concentrated, a separation operation was then performed using water/ethyl acetate, and the product was purified by silica gel column chromatography (developing solvent: hexane/ethyl acetate = 95/5), yielding 9.89 g of a compound 11a in the form of a white solid (yield: 79%). The identification data for the compound 11a are shown below.

¹H-NMR (CDCl₃): δ 7.2 to 6.6 (m, 12H), 6.2 to 5.9 (m, 4H), 5.5 to 5.1 (m, 8H), 4.6 to 4.4 (m, 8H), 2.6 (m, 4H), 2.2 (s, 6H), 2.1 (s, 6H), 1.8 (m, 4H), 1.6 (m, 4H), 1.4 (m, 2H), 1.2 (m, 4H), 0.5 (s, 6H)

### <Synthesis of Compound 1-11>

First, 2.0 g (2.5 mmol) of the compound 11a was weighed into a three-neck flask, and then dissolved in 5 mL of dichloromethane. Subsequently, 2 mg (0.03 mmol) of aniline and 6.7 g (30 mmol) of 1,1,1,3,3,5,5-heptamethyltrisiloxane were added and stirred. Next, 0.3 g (0.03 mmol) of a toluene solution of platinum/1,3-divinyl-1,1,3,3-tetramethyldisiloxane complex (platinum content: 3% by mass) was added, and the resulting mixture was stirred at 25°C for 24 hours. Following completion of the reaction, the solvent was removed by distillation under reduced pressure, and the resulting product was purified by silica gel column chromatography (developing solvent: hexane/ethyl acetate = 99/1), yielding 1.6 g of the compound 1-11 (yield: 40%). The identification data for the compound 1-11 are shown below.

¹H-NMR (CDCl₃): δ 7.2 to 6.5 (m, 12H), 4.0 to 3.8 (m, 8H), 2.7 to 2.5 (m, 4H), 2.2 (s, 6H), 2.1 (s, 6H), 1.9 to 1.7 (m, 12H), 1.6 to 1.5 (m, 4H), 1.4 to 1.3 (m, 2H), 1.3 to 1.1 (m, 4H), 1.0 to 0.8 (m, 8H), 0.5 (s, 6H), 0.2 to 0.0 (m, 84H)

### (Example 12)

A compound 1-12 was produced using the procedure described below.

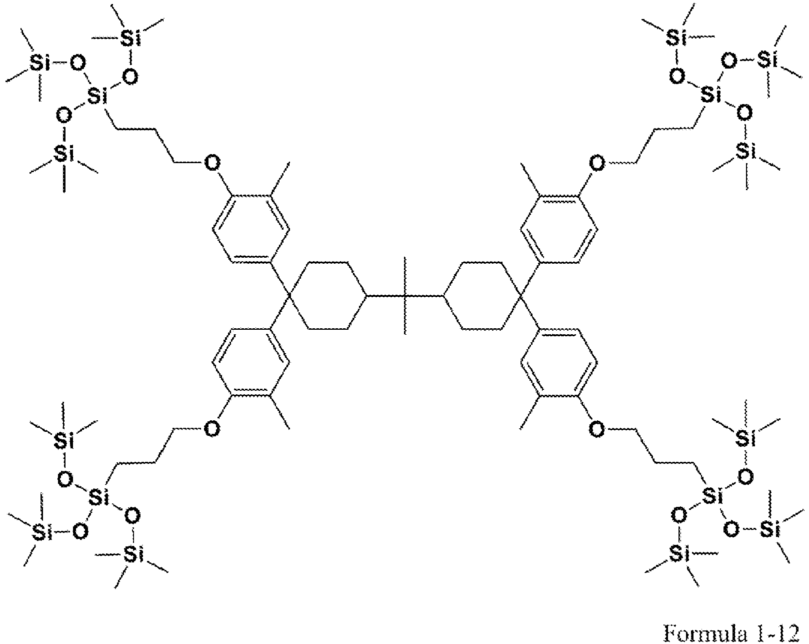

First, 5.0 g (6.3 mmol) of the compound 11a was weighed into a three-neck flask, and then dissolved in 10 mL of dichloromethane. Subsequently, 6 mg (0.06 mmol) of aniline and 23 g (75 mmol) of tris(trimethylsilyloxy)silane were added and stirred. Next, 0.80 g (0.06 mmol) of a toluene solution of platinum/1,3-divinyl-1,1,3,3-tetramethyldisiloxane complex (platinum content: 3% by mass) was added, and the resulting mixture was stirred at 25°C for 24 hours. Following completion of the reaction, the solvent was removed by distillation under reduced pressure, the resulting product was purified by silica gel column chromatography (developing solvent: hexane/ethyl acetate = 99/1), and the solvent was removed under reduced pressure, yielding 5.4 g of the compound 1-12 (yield: 43%). The identification data for the compound 1-12 are shown below.

¹H-NMR (CDCl₃): δ 7.2 to 6.5 (m, 12H), 4.0 to 3.8 (m, 8H), 2.7 to 2.5 (m, 4H), 2.2 (s, 6H), 2.1 (s, 6H), 1.9 to 1.7 (m, 12H), 1.6 to 1.5 (m, 4H), 1.4 to 1.3 (m, 2H), 1.3 to 1.1 (m, 4H), 0.7 to 0.5 (m, 8H), 0.5 (s, 6H), 0.10 (s, 54H), 0.09 (s, 54H)

### (Example 13)

A compound 1-13 was produced using the procedure described below.

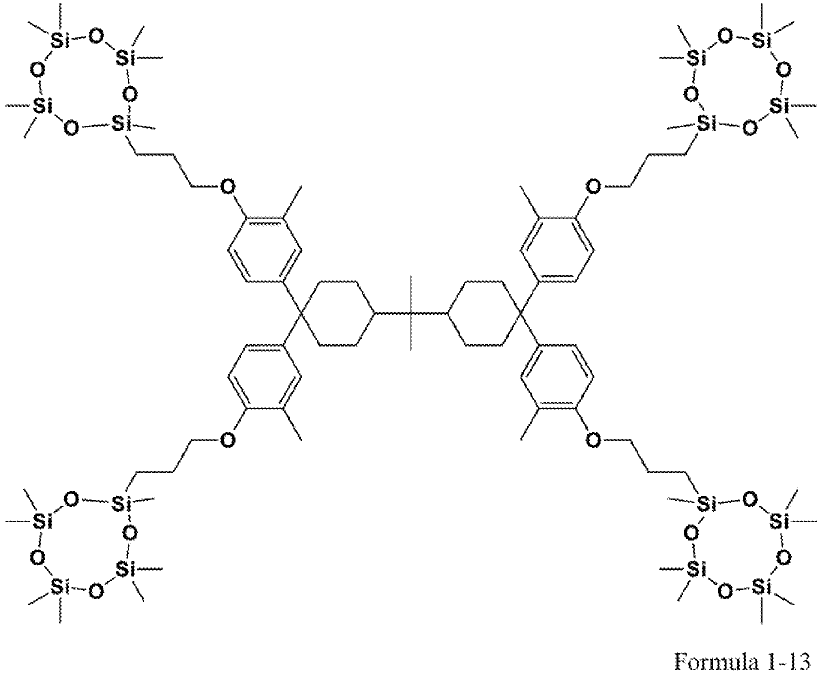

First, 2.0 g (2.5 mmol) of the compound 11a was weighed into a three-neck flask, and then dissolved in 5 mL of dichloromethane. Subsequently, 2 mg (0.03 mmol) of aniline and 8.5 g (30 mmol) of heptamethylcyclotetrasiloxane were added and stirred. Next, 0.3 g (0.03 mmol) of a toluene solution of platinum/1,3-divinyl-1,1,3,3-tetramethyldisiloxane complex (platinum content: 3% by mass) was added, and the resulting mixture was stirred at 25°C for 24 hours. Following completion of the reaction, the solvent was removed by distillation under reduced pressure, the resulting product was purified by silica gel column chromatography (developing solvent: hexane/ethyl acetate = 99/1), and the solvent was removed under reduced pressure, yielding 2.3 g of the compound 1-13 (yield: 48%). The identification data for the compound 1-13 are shown below.

¹H-NMR (CDCl₃): δ 7.2 to 6.5 (m, 12H), 4.0 to 3.8 (m, 8H), 2.7 to 2.5 (m, 4H), 2.2 (s, 6H), 2.1 (s, 6H), 1.9 to 1.7 (m, 12H), 1.6 to 1.5 (m, 4H), 1.4 to 1.3 (m, 2H), 1.3 to 1.1 (m, 4H), 1.0 to 0.8 (m, 8H), 0.5 (s, 6H), 0.2 to 0.0 (m, 84H)

### (Example 14)

A compound 1-14 was produced using the procedure described below.

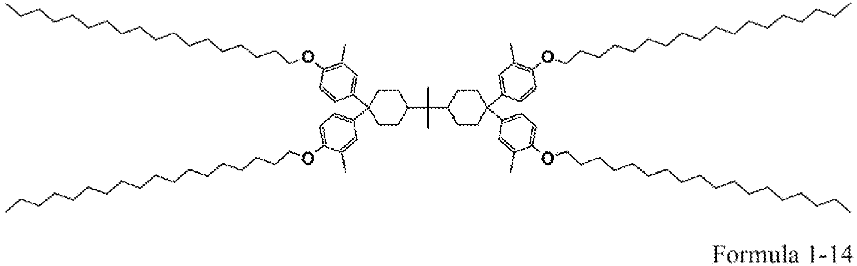

A three-neck flask was charged with a mixture of 5.0 g (7.9 mmol) of tekOC-4HBPA, 14.6 g (63 mmol) of potassium carbonate, 15.8 g (47 mmol) of 1-bromooctane and 40 mL of N,N-dimethylformamide, and the resulting mixture was stirred under a nitrogen atmosphere at 80°C for 6 hours. Following separation of the solids by filtration, a separation operation was performed using water/ethyl acetate, and the product was purified by silica gel column chromatography (developing solvent: hexane/ethyl acetate = 99/1), yielding 5.2 g of the compound 1-14 (yield: 40%). The identification data for the compound 1-14 are shown below.

¹H-NMR (CDCl₃): δ 7.2 to 6.5 (m, 12H), 4.0 to 3.8 (m, 8H), 2.7 to 2.5 (m, 4H), 2.2 (s, 6H), 2.1 (s, 6H), 1.9 to 1.7 (m, 12H), 1.6 to 1.1 (m, 130H), 0.8 to 0.5 (m, 18H)

### (Example 15)

A compound 1-15 was produced using the procedure described below.

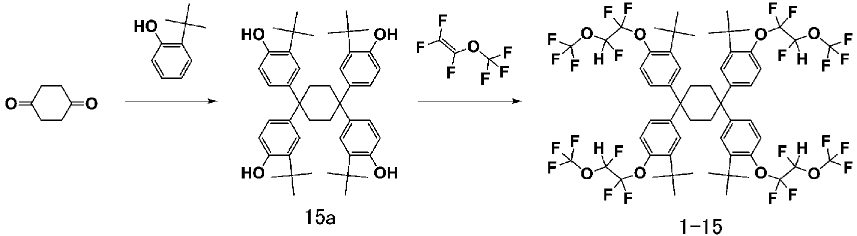

### <Synthesis of Compound 15a>

A three-neck flask was charged with 128.6 g (856 mmol) of 2-tert-butylphenol, 8.0 g (71.3 mmol) of cyclohexanone and 1.9 g (9.4 mmol) of 1-octanethiol, the temperature was raised to 50°C, and hydrogen chloride gas was then bubbled through the reaction mixture for 2 hours. Following completion of the hydrogen chloride gas bubbling, the reaction mixture was stirred at 50°C for 12 hours. Following completion of the reaction, hydrogen chloride gas was removed from the reaction vessel by bubbling nitrogen gas through the reaction mixture, and excess 2-tert-butylphenol was removed by distillation under reduced pressure. The thus obtained crude product was purified by silica gel column chromatography (hexane/ethyl acetate), yielding 40 g of a compound 15a (yield: 83%). The identification data for the compound 15a are shown below.

¹H-NMR (Acetone-d6): δ 7.2 to 6.6 (m, 12H), 4.5 (br, 4H), 2.2 (s, 8H), 1.4 (s, 36H)

### <Synthesis of Compound 1-15>

First, 2.4 g (3.5 mmol) of the compound 15a was weighed into a 100 mL two-neck round-bottom flask and dissolved in a mixed solvent containing 14 mL of DMF and 3.5 mL of 2-propanol. Subsequently, 2.4 g (17 mmol) of potassium carbonate was added and stirred to form a suspension. The flask openings were sealed with septa, perfluoromethyl vinyl ether that had been transferred into a 5 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 3.1 g of the compound 1-15 (yield: 65%). The identification data for the compound 1-15 are shown below.
¹H-NMR (CDCl₃): δ 7.2 to 6.6 (m, 12H), 6.1 to 5.8 (m, 4H), 2.2 (s, 8H), 1.4 (s, 36H)
¹⁹F-NMR (CDCl₃): δ -57 (s, 12F), -82 (m, 8F), -142 (m, 4F)

### (Example 16)

A compound 1-16 was produced using the procedure described below.

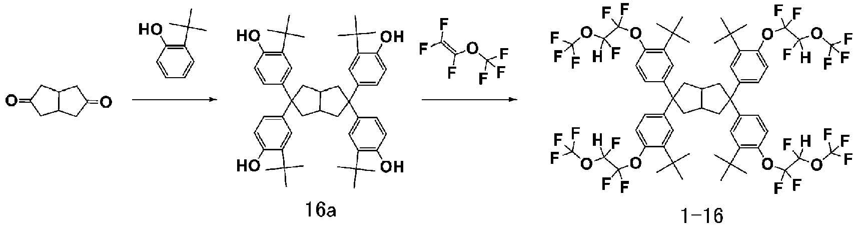

### <Synthesis of Compound 16a>

A three-neck flask was charged with 42.9 g (285 mmol) of 2-tert-butylphenol, 3.3 g (23.8 mmol) of tetrahydropentalene-2,5-dione and 0.63 g (3.1mmol) of 1-octanethiol, the temperature was raised to 50°C, and hydrogen chloride gas was then bubbled through the reaction mixture for 2 hours. Following completion of the hydrogen chloride gas bubbling, the reaction mixture was stirred at 50°C for 12 hours. Following completion of the reaction, hydrogen chloride gas was removed from the reaction vessel by bubbling nitrogen gas through the reaction mixture, and excess 2-tert-butylphenol was removed by distillation under reduced pressure. The thus obtained crude product was purified by silica gel column chromatography (hexane/ethyl acetate), yielding 13 g of a compound 16a (yield: 79%). The identification data for the compound 16a are shown below.

¹H-NMR (Acetone-d6): δ 7.2 to 6.7 (m, 12H), 4.5 (br, 4H), 2.4 to 1.7 (m, 10H), 1.4 (s, 36H)

### <Synthesis of Compound 1-16>

First, 2.5 g (3.5 mmol) of the compound 16a was weighed into a 100 mL two-neck round-bottom flask and dissolved in a mixed solvent containing 14 mL of DMF and 3.5 mL of 2-propanol. Subsequently, 2.4 g (17 mmol) of potassium carbonate was added and stirred to form a suspension. The flask openings were sealed with septa, perfluoromethyl vinyl ether that had been transferred into a 5 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 3.2 g of the compound 1-16 (yield: 67%). The identification data for the compound 1-16 are shown below.
¹H-NMR (CDCl₃): δ 7.2 to 6.7 (m, 12H), 6.1 to 5.8 (m, 4H), 2.4 to 1.7 (s, 10H), 1.4 (s, 36H)
¹⁹F-NMR (CDCl₃): δ -57 (s, 12F), -82 (m, 8F), -142 (m, 4F)

### (Example 17)

A compound 1-17 was produced using the procedure described below.

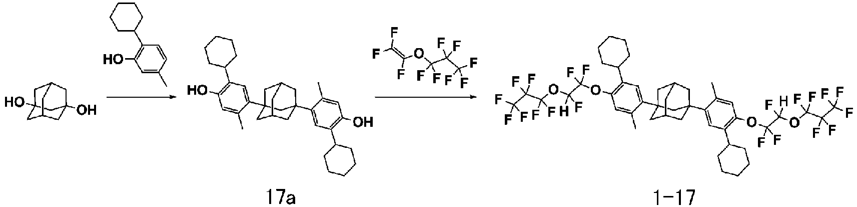

### <Synthesis of Compound 17a>

A three-neck flask was charged with 3.8 g (20 mmol) of 2-cyclohexyl-5-methylphenol, 2.5 g (15 mmol) of 1,3-adamandiol and 1.0 g (10 mmol) of concentrated sulfuric acid, and the resulting mixture was stirred at 90°C for 3 hours. Following completion of the reaction, the reaction mixture was cooled by standing at 25°C, 40 g of acetone was added, a sodium hydroxide aqueous solution was added at 0°C to adjust the pH to 7, and the mixture was stirred for one hour. Following a separation operation using water/ethyl acetate, the product was purified by silica gel column chromatography (developing solvent: hexane/ethyl acetate = 95/5), yielding 5.0 g of a compound 17a (yield: 65%). The identification data for the compound 17a are shown below.

¹H-NMR (acetone-d6): δ 7.1 (s, 2H), 7.0 (s, 2H), 4.5 (br, 2H), 2.9 (m, 2H), 2.6 (s, 2H), 2.3 (m, 7H), 2.2 to 1.4 (m, 31H)

### <Synthesis of Compound 1-17>

First, 1.8 g (3.5 mmol) of the compound 17a was weighed into a 100 mL two-neck round-bottom flask and dissolved in a mixed solvent containing 14 mL of DMF and 3.5 mL of 2-propanol. Subsequently, 2.4 g (17 mmol) of potassium carbonate was added and stirred to form a suspension. Next, 7.1 mL (42 mmol) of perfluoropropoxyethylene was added, and the resulting mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 1.8 g of the compound 1-17 (yield: 60%). The identification data for the compound 1-17 are shown below.
¹H-NMR (CDCl₃): δ 7.1 (s, 2H), 7.0 (s, 2H), 6.1 to 5.8 (m, 2H), 2.9 (m, 2H), 2.6 (s, 2H), 2.3 (m, 7H), 2.2 to 1.4 (m, 31H)
¹⁹F-NMR (CDCl₃): δ -79 (m, 6F), -81 to -85 (m, 8F), -127 (s, 4F), -140 (m, 2F)

### (Example 18)

A compound 3-1 was produced using the procedure described below.

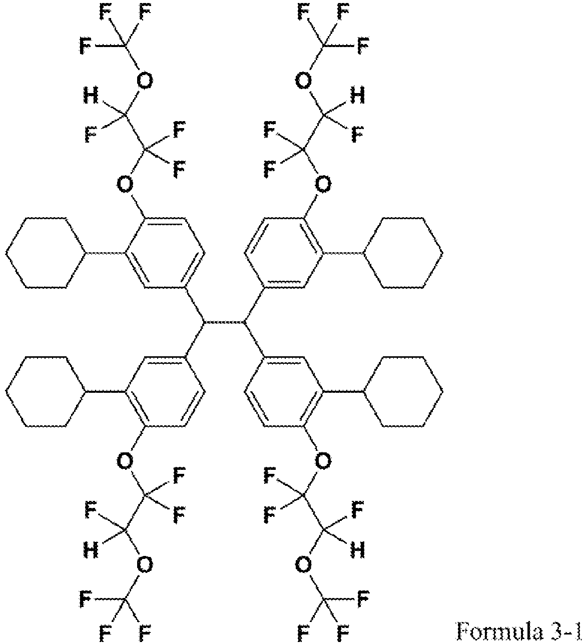

First, 1.2 g (1.7 mmol) of 4,4',4",4"'-(1,2-ethanediylidene)tetrakis[2-cyclohexylphenol] was weighed into a 50 mL two-neck round-bottom flask and dissolved in a mixed solvent containing 7 mL of DMF and 2 mL of 2-propanol. Subsequently, 1.1 g (8 mmol) of potassium carbonate was added and stirred to form a suspension. The flask openings were sealed with septa, perfluoromethyl vinyl ether that had been transferred into a 3 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 1.5 g of the compound 3-1 in the form of a colorless glass-like solid (yield: 65%). The identification data for the compound 3-1 are shown below.
¹H-NMR (CDCl₃): δ 7.1 to 6.8 (m, 12H), 6.1 to 5.8 (m, 4H), 4.5 (s, 2H), 2.7 (m, 4H), 1.7 to 1.2 (m, 40H)
¹⁹F-NMR (CDCl₃): δ -57 (s, 12F), -82 (m, 8F), -142 (m, 4F)

### (Example 19)

A compound 2-3 was produced using the procedure described below.

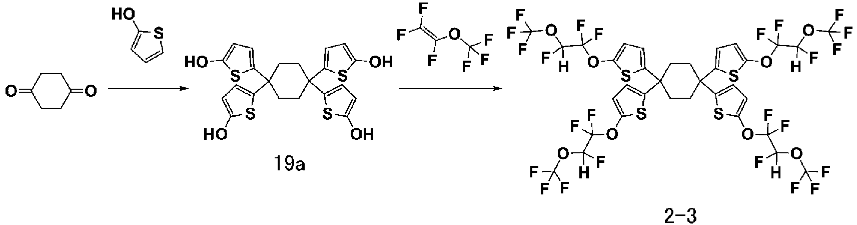

A three-neck flask was charged with 4.3 g (43 mmol) of 2-hydroxythiophene, 0.40 g (3.6 mmol) of cyclohexanone and 0.1 g (0.5 mmol) of 1-octanethiol, the temperature was raised to 50°C, and hydrogen chloride gas was then bubbled through the reaction mixture for 2 hours. Following completion of the hydrogen chloride gas bubbling, the reaction mixture was stirred at 50°C for 12 hours. Following completion of the reaction, hydrogen chloride gas was removed from the reaction vessel by bubbling nitrogen gas through the reaction mixture, and excess 2-hydroxythiophene was removed by distillation under reduced pressure, yielding 1.8 g of a crude product of a compound 19a. This crude product was used, as is, in the subsequent step.

Subsequently, 1.8 g of the crude product of the compound 19a was weighed into a 100 mL two-neck round-bottom flask and dissolved in a mixed solvent containing 14 mL of DMF and 3.5 mL of 2-propanol. Subsequently, 2.4 g (17 mmol) of potassium carbonate was added and stirred to form a suspension. The flask openings were sealed with septa, perfluoromethyl vinyl ether that had been transferred into a 5 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 1.4 g of the compound 1-19 (yield: 35%). The identification data for the compound 1-19 are shown below.
¹H-NMR (CDCl₃): δ 6.5 to 6.4 (m, 8H), 6.0 to 5.7(m, 4H), 2.2 (s, 8H)
¹⁹F-NMR (CDCl₃): δ -59 (s, 12F), -84 (m, 8F), -144 (m, 4F)

### (Example 20)

A compound 1-18 was produced using the procedure described below.

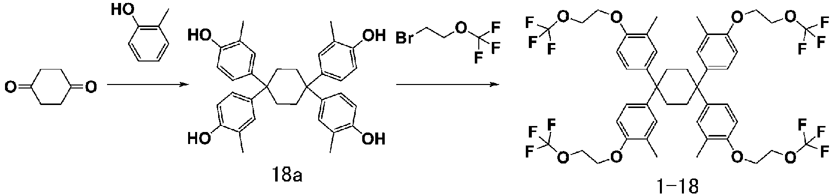

### <Synthesis of Compound 18a>

A three-neck flask was charged with 43 g (400 mmol) of 2-methylphenol, 5.6 g (50 mmol) of cyclohexanone and 1.0 g (5 mmol) of 1-octanethiol, the temperature was raised to 50°C, and hydrogen chloride gas was then bubbled through the reaction mixture for 2 hours. Following completion of the hydrogen chloride gas bubbling, the reaction mixture was stirred at 50°C for 12 hours. Following completion of the reaction, hydrogen chloride gas was removed from the reaction vessel by bubbling nitrogen gas through the reaction mixture, and excess 2-methylphenol was removed by distillation under reduced pressure. The thus obtained crude product was purified by silica gel column chromatography (hexane/ethyl acetate) yielding 22 g of a compound 18a (yield: 85%). The identification data for the compound 18a are shown below.

¹H-NMR (acetone-d6): δ 7.2 to 6.6 (m, 12H), 4.5 (br, 4H), 2.2 (s, 8H), 2.1 (s, 12H)

### <Synthesis of Compound 1-18>

First, 0.82 g (1.6 mmol) of the compound 18a was weighed into a 50 mL two-neck round-bottom flask and dissolved in 8 mL of DMF. Subsequently, 6.3 g (19 mmol) of cesium carbonate and 3.7 g (19 mmol) of 1-bromo-3-(trifluoromethoxy)propane were added, and the resulting mixture was stirred at 70°C for 12 hours. Following a separation operation using water/ethyl acetate, the product was purified by silica gel column chromatography (developing solvent: hexane/ethyl acetate = 95/5), and the solvent was then removed under reduced pressure, yielding 0.61 g of the compound 1-18 (yield: 40%). The identification data for the compound 1-18 are shown below.
¹H-NMR (CDCl₃): δ 7.2 to 6.6 (m, 12H), 4.4 to 4.1 (m, 8H), 4.0 to 3.7 (m, 8H), 2.2 (s, 8H), 2.1 (s, 12H)
¹⁹F-NMR (CDCl₃): δ -59 (s, 12F)

### (Example 21)

A compound 4 was produced using the procedure described below.

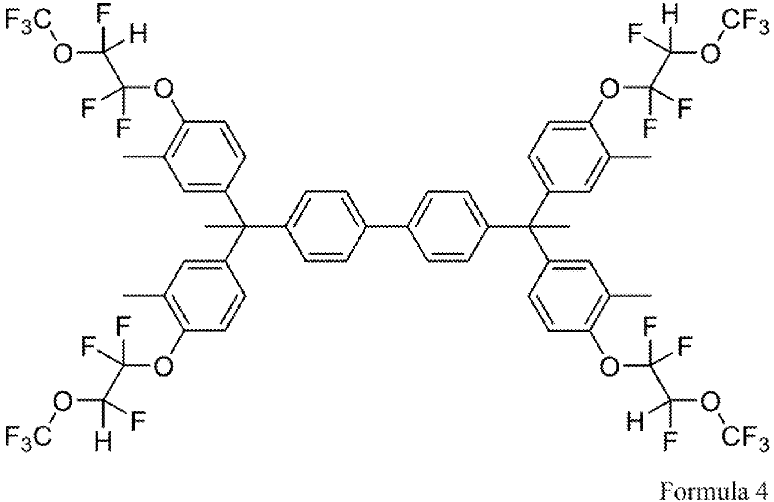

First, 3.0 g (3.5 mmol) of α,α,α',α'-tetrakis(4-hydroxyphenyl)-4,4'-diethylbiphenyl (manufactured by Honshu Chemical Industry Co., Ltd., hereinafter also abbreviated as "tekOC-DABP") was weighed into a 100 mL two-neck round-bottom flask and dissolved in a mixed solvent containing 14 mL of DMF and 4.0 mL of 2-propanol. Subsequently, 2.6 g (19 mmol) of potassium carbonate was added and stirred to form a suspension. The flask openings were sealed with septa, perfluoromethyl vinyl ether that had been transferred into a 5 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 4.10 g of the compound 4 in the form of a colorless glass-like solid (yield: 89%).

### (Example 22)

A compound 5 was produced using the procedure described below.

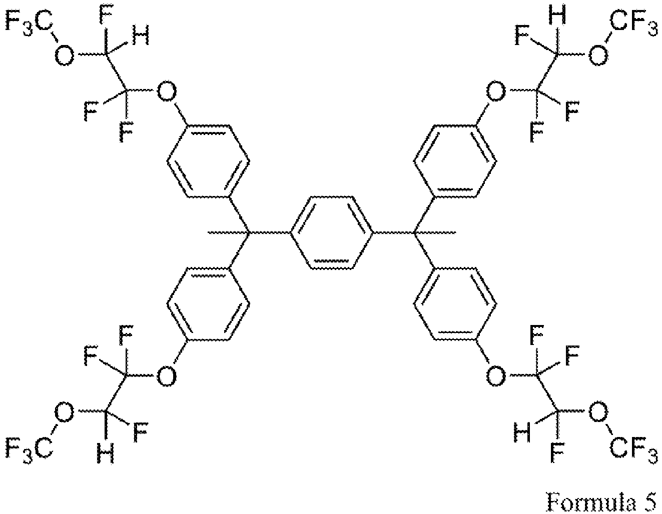

First, 2.0 g (4.0 mmol) of α,α,α',α'-tetrakis(4-hydroxyphenyl)-1,4-diethylbenzene (manufactured by Honshu Chemical Industry Co., Ltd., hereinafter also abbreviated as "tekP-DAB") was weighed into a 100 mL two-neck round-bottom flask and dissolved in a mixed solvent containing 16 mL of DMF and 4.0 mL of 2-propanol. Subsequently, 2.9 g (21 mmol) of potassium carbonate was added and stirred to form a suspension. The flask openings were sealed with septa, perfluoromethyl vinyl ether that had been transferred into a 5 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 95/5), and the solvent was then removed under reduced pressure, yielding 4.60 g of the compound 5 in the form of a colorless glass-like solid (yield: 98%).

### (Example 23)

A compound 6 was produced using the procedure described below.

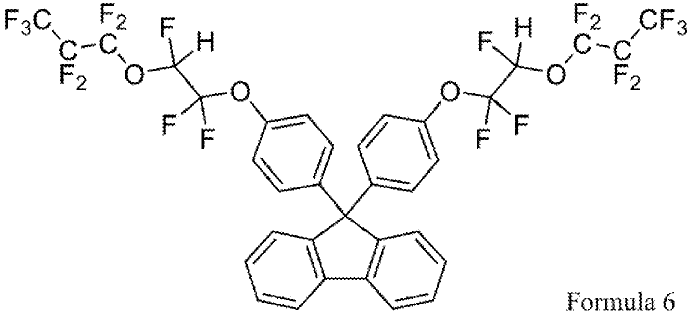

First, 1.00 g (2.85 mmol) of 9,9-bis(4-hydroxyphenyl)fluorene (manufactured by Tokyo Chemical Industry Co., Ltd.) was weighed into a 100 mL two-neck round-bottom flask and dissolved in a mixed solvent containing 12 mL of DMF and 3 mL of 2-propanol. Subsequently, 0.99 g (7.16 mmol) of potassium carbonate was added and stirred to form a suspension. Next, 2.5 mL (15 mmol) of perfluoropropoxyethylene was added, and the resulting mixture was stirred at 25°C for 12 hours. Following a separation operation using water/ethyl acetate, the product was purified by silica gel column chromatography (developing solvent: hexane/ethyl acetate = 9/1), and the solvent was then removed under reduced pressure, yielding 1.76 g of the compound 6 in the form of a white solid (yield: 70%).

### (Example 24)

A compound 7 was produced using the procedure described below.

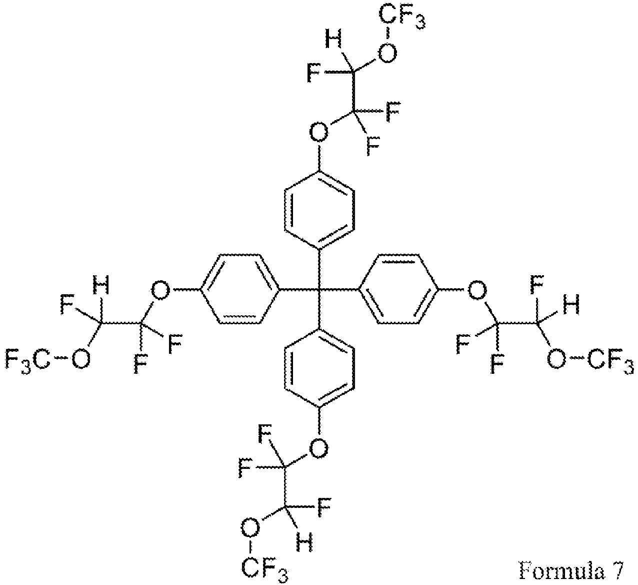

First, 0.7 g (1.73 mmol) of 4,4',4",4"'-methanetetrayltetraphenol (manufactured by Tokyo Chemical Industry Co., Ltd.) was weighed into a 50 mL two-neck round-bottom flask and dissolved in a mixed solvent containing 7 mL of DMF and 2.0 mL of 2-propanol. Subsequently, 1.19 g (8.6 mmol) of potassium carbonate was added and stirred to form a suspension. The flask openings were sealed with septa, perfluoromethyl vinyl ether that had been transferred into a 3 L Tedlar bag was connected via a syringe, and the reaction mixture was stirred at 25°C for 12 hours. Following a separation operation using water/ethyl acetate, the product was purified by silica gel column chromatography (developing solvent: hexane/ethyl acetate = 9/1), and the solvent was then removed under reduced pressure, yielding 1.51 g of the compound 7 in the form of a colorless glass-like solid (yield: 79%).

### (Example 25)

A compound 8 was produced in accordance with the procedure disclosed in Example 1 of International Patent Publication No. 2022/034907.

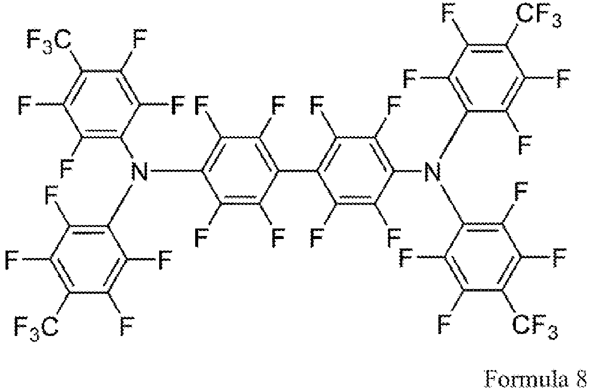

### [Evaluations]

The compound of each example was subjected to the following evaluations.

### (Evaluation of Deposition Properties)

Using a vacuum differential thermobalance (VPE-9000, manufactured by Advance RIKO, Inc.), the heat mass loss of the compound was measured under vacuum. Specifically, 50 mg of the compound was placed in a cell with an internal diameter of 7 mm, and the mass loss of the compound was evaluated when the temperature was raised from 50°C to 500°C at a rate of 2°C per minute under a vacuum of 1×10⁻³ Pa or lower. Compounds that exhibited a mass loss of 95% or more in the range from 150°C to 300°C were classified as "depositable," whereas compounds that did not meet this condition were classified as "not depositable."

The results revealed that all of the compounds of Examples 1 to 25 were "depositable."

### (Production of Substrate with Organic Film)

Using a deposition apparatus, a quartz substrate having an organic film containing the compound (hereinafter also referred to as a "substrate with an organic film") was produced by depositing the compound onto the surface of a 25 mm × 25 mm × 0.525 mm quartz substrate via a metal mask with a 25 mm × 12 mm opening. Deposition of the compound was conducted under conditions including a deposition rate of 0.1 nm/second. The deposition of the compound was continued until the film thickness reached 50 nm as measured by the film thickness meter included with the deposition apparatus.

### (Evaluation of Film Formability: Measurement of Total Light Transmittance and Haze)

For each substrate with an organic film described above, the total light transmittance and the haze of the portion where the organic film had been deposited were measured using a haze meter (model number: HZ-V3, manufactured by Suga Test Instruments Co., Ltd., measurement light: D65 light). The results are shown in Tables 1 to 3.

In Examples 1 to 20, the total light transmittance of the portion where the organic film had been deposited was 93% or higher, and the haze was 1.0 or lower. These results confirmed that the deposited films formed from the compounds of Examples 1 to 20 were colorless, transparent, and uniform films.

In contrast, in Examples 23 and 24, the haze of the portion where the organic film had been deposited exceeded 1.0, confirming that the deposited films were nonuniform films.

### (Ag Deposition)

Using a deposition apparatus and without using a mask, Ag was deposited across the entire surface of the organic film side of the above substrate with an organic film. The Ag deposition was conducted under conditions including a deposition rate of 0.05 nm/second. Further, the Ag deposition was continued until the film thickness reached 70 nm, as measured by the film thickness meter included with the deposition apparatus.

### (Evaluation of Ag Repellency: Measurement of Transmittance)

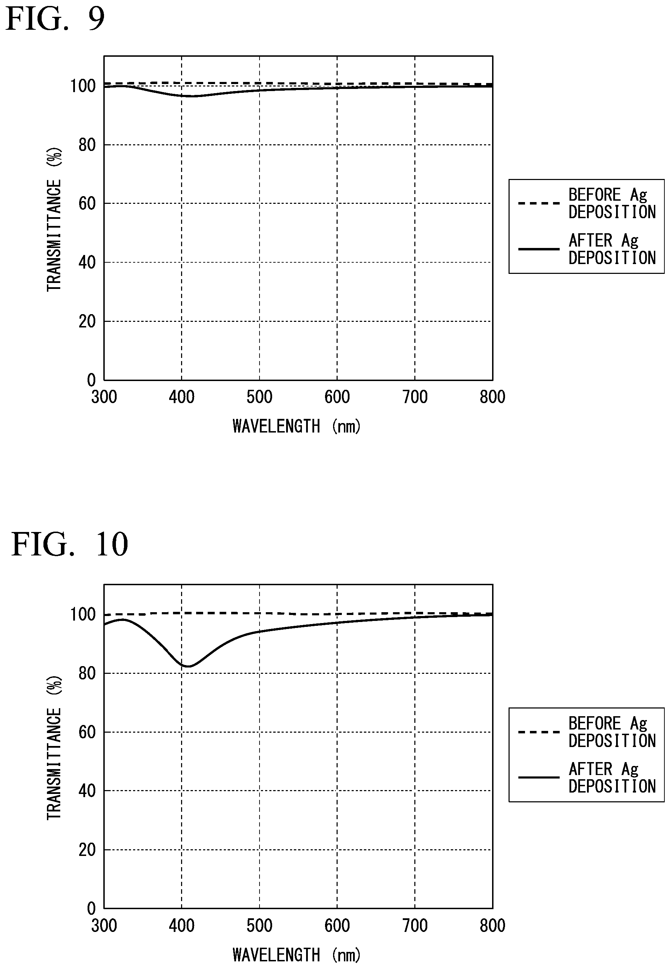

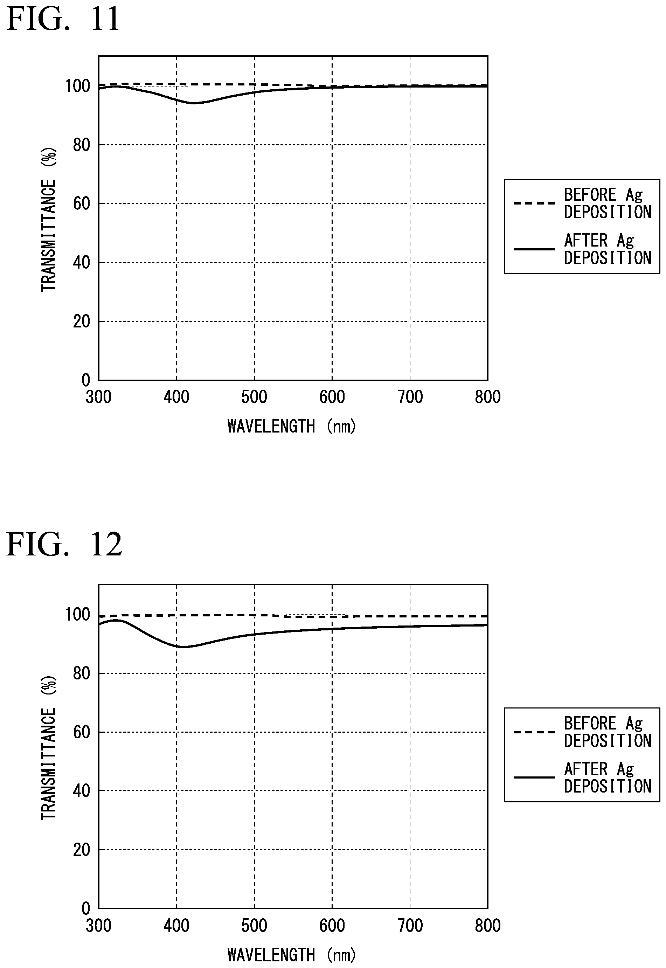

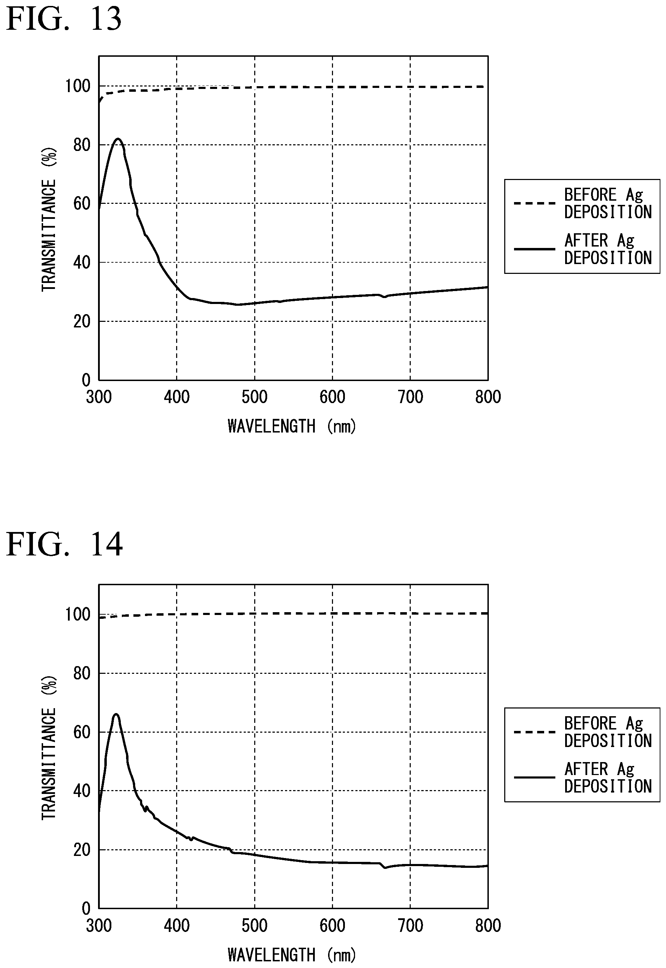

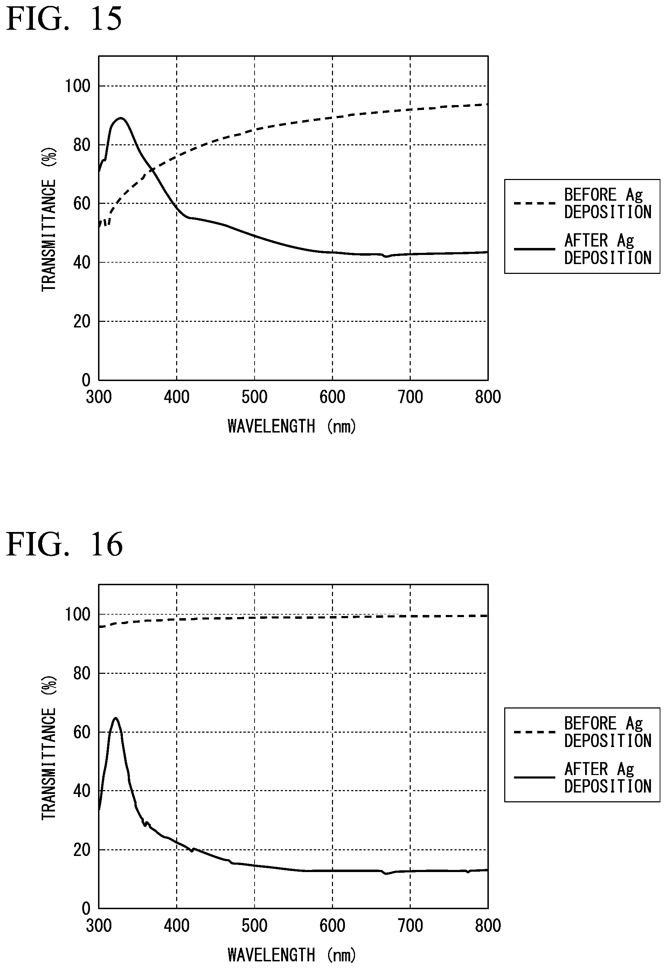

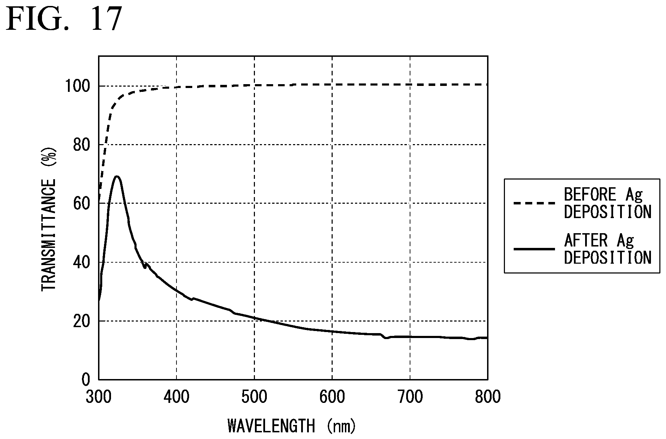

For each substrate with an organic film, before and after Ag deposition, the transmittance of the portion where the organic film had been deposited within a wavelength range from 300 to 800 nm was measured using a spectrophotometer (model: UV-3600 Plus, manufactured by Shimadzu Corporation), with a quartz substrate used as reference. Tables 1 to 3 show the transmittance at a wavelength of 436 nm for each example. FIGS. 9 to 17 show the transmittance spectra for the wavelength range from 300 to 800 nm for Examples 1 to 4 and 21 to 25, respectively.

In Examples 1 to 20, even after the Ag deposition, the transmittance at a wavelength of 436 nm in the portion where the organic film had been deposited was 55% or higher, indicating excellent transmittance of blue light. In Examples 1 to 6, 9, 15 to 17 and 19, the transmittance was 65% or higher, indicating even more superior transmittance of blue light. Examples 1 to 4 and 6 exhibited transmittance of 80% or higher, indicating particularly superior transmittance of blue light. These results confirmed that the deposited films formed from the compounds of Examples 1-20 all exhibit excellent Ag repellency. The deposited films formed from the compounds of Examples 1 to 6, 9, 15 to 17 and 19 exhibited even better Ag repellency. The deposited films formed from the compounds of Examples 1 to 4 and 6 exhibited particularly superior Ag repellency.

In contrast, in Examples 21 to 25, following Ag deposition, the transmittance at a wavelength of 436 nm in the portion where the organic film had been deposited was less than 55%.

**[Table 1]**

| Example | 1 | 2 | 3 | 4 | 5 | 6 | 7 | 8 | 9 |
|---|---|---|---|---|---|---|---|---|---|
| Total light transmittance (%) | 94.0 | 93.8 | 93.9 | 93.3 | 94.0 | 94.0 | 93.3 | 93.2 | 93.2 |
| Haze | 0.10 | 0.15 | 0.81 | 0.09 | 0.11 | 0.08 | 0.20 | 0.18 | 0.16 |
| Transmittance before Ag deposition (% @ 436 nm) | 100.0 | 100.0 | 100.0 | 99.4 | 99.7 | 100.0 | 99.6 | 99.7 | 99.5 |
| Transmittance after Ag deposition (% @ 436 nm) | 96.8 | 86.5 | 94.5 | 90.0 | 69.2 | 90.8 | 56.4 | 60.2 | 75.2 |

**[Table 2]**

| Example | 10 | 11 | 12 | 13 | 14 | 15 | 16 | 17 | 18 | 19 |
|---|---|---|---|---|---|---|---|---|---|---|
| Total light transmittance (%) | 93.1 | 93.7 | 93.6 | 93.6 | 93.0 | 93.2 | 93.1 | 93.3 | 93.2 | 93.1 |
| Haze | 0.30 | 0.41 | 0.44 | 0.40 | 0.75 | 0.23 | 0.20 | 0.56 | 0.22 | 0.18 |
| Transmittance before Ag deposition (% @ 436 nm) | 99.3 | 99.7 | 99.8 | 99.6 | 99.0 | 99.4 | 99.5 | 99.8 | 99.6 | 99.4 |
| Transmittance after Ag deposition (% @ 436 nm) | 60.2 | 55.2 | 57.4 | 55.1 | 55.1 | 65.4 | 67.5 | 66.5 | 55.0 | 65.1 |

**[Table 3]**

| Example | 20 | 21 | 22 | 23 | 24 | 25 |
|---|---|---|---|---|---|---|
| Total light transmittance (%) | 93.1 | 93.7 | 93.5 | 99.3 | 93.6 | 93.7 |
| Haze | 0.21 | 0.12 | 0.23 | 13.7 | 1.11 | 0.14 |
| Transmittance before Ag deposition (% @ 436 nm) | 99.2 | 98.9 | 99.5 | 80.2 | 98.5 | 99.8 |
| Transmittance after Ag deposition (% @ 436 nm) | 55.7 | 26.6 | 22.4 | 54.2 | 18.7 | 26.3 |

### [Reference Signs List]

1, 2, 3 Conductive film
10, 13, 116 First film
10A, 13A First region
10B, 13B Second region
10X Conductive material
15, 117 Second film
20, 118 Third film
20a, 50a Surface
50 Substrate
110 Substrate
111 Anode
115 Cathode
P Compound
M Mask

## Claims

1. A compound having a molecular weight within a range from 500 to 5,000,
composed of hydrogen atoms, carbon atoms and hetero atoms, wherein the hetero atoms include at least one type of hetero atom selected from among an oxygen atom, sulfur atom, fluorine atom and silicon atom, and
having at least one alicyclic structure, at least one aromatic ring structure bonded directly to the alicyclic structure, and at least one monovalent substituent Ms bonded directly to the aromatic ring structure, wherein
the monovalent substituent Ms is at least one type of group selected from among monovalent groups containing a fluorine-containing aliphatic group, monovalent groups containing a fluorinated sulfur group, monovalent groups containing a siloxanyl group, and monovalent groups containing a saturated hydrocarbon group.

2. The compound according to Claim 1, wherein a number of the alicyclic structures is two or more.

3. The compound according to Claim 1, wherein the alicyclic structure is monocyclic.

4. The compound according to Claim 1, wherein the monovalent group containing a fluorine-containing aliphatic group is represented by formula f1 shown below:
-Z^{l}-(O-R¹)ₙ-F Formula f1
wherein R¹ represents an alkylene group or a fluoroalkylene group, n represents an integer of 12 or greater, the R¹ groups are identical or different, and Z¹ represents a single bond or a divalent linking group that does not contain an aromatic ring structure.

5. The compound according to Claim 1, wherein the monovalent group containing a fluorine-containing aliphatic group is at least one group selected from among groups represented by formula f5 and formula f6 shown below:
-Z²-(-X¹-Rf¹)ₙ₁ Formula f5
-Z³-(-X²-Rf²-R²)n₂ Formula f6
wherein Rf¹ represents -CF₃, -CF₂H or -CFH₂, Rf² represents -CF₂- or -CFH-, R² represents an alkyl group, X¹ and X² each represent -O- or -S-, Z² and Z³ each represent a single bond, or a (n1+1)-valent or (n2+1)-valent linking group that does not contain an aromatic ring structure, and n1 and n2 each represent an integer within a range from 1 to 9.

6. The compound according to Claim 1, wherein the monovalent group containing a fluorinated sulfur group is at least one group selected from among groups represented by formula fs1 and formula fs2 shown below:
-Z⁴-SF₅ Formula fs1
-Z⁵-SF₄-R³ Formula fs2
wherein R³ represents a hydrocarbon group, and Z⁴ and Z⁵ each represent a single bond or a divalent linking group that does not contain an aromatic ring structure.

7. The compound according to Claim 1, wherein the monovalent group containing a siloxanyl group is at least one group selected from among groups represented by formula sx1 and formula sx2 shown below: 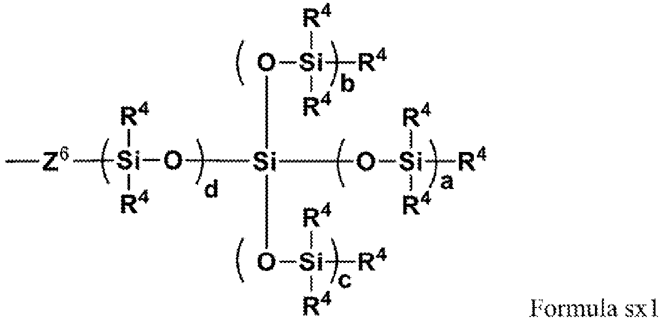 wherein each R⁴ independently represents an alkyl group of 1 to 10 carbon atoms or an aryl group of 6 to 12 carbon atoms, Z⁶ represents a single bond or a divalent linking group that does not contain an aromatic ring structure, and each of a, b, c and d independently represents an integer within a range from 0 to 20, provided that a+b+c+d > 0, 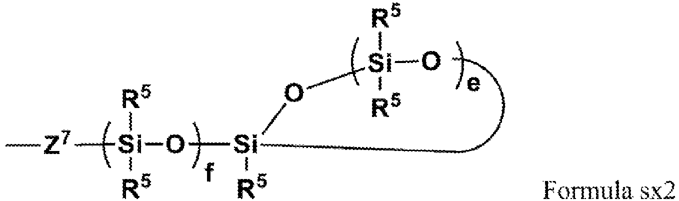 wherein each R⁵ independently represents an alkyl group of 1 to 10 carbon atoms or an aryl group of 6 to 12 carbon atoms, Z⁷ represents a single bond or a divalent linking group that does not contain an aromatic ring structure, e represents an integer within a range from 1 to 20, and f represents an integer within a range from 0 to 20, provided that e+f > 1.

8. The compound according to Claim 1, wherein the monovalent group containing a saturated hydrocarbon group is represented by formula sh1 shown below:
-Z⁸-R⁶ Formula sh1
wherein R⁶ represents a saturated hydrocarbon group of 4 to 24 carbon atoms, and Z⁸ represents a single bond, a divalent linking group that does not contain an aromatic ring structure, an oxygen atom, or a sulfur atom.

9. The compound according to Claim 1, represented by formula 1 shown below: 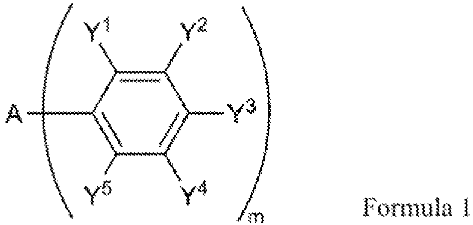 wherein m represents an integer within a range from 1 to 8, A represents an m-valent group containing the at least one alicyclic structure, Y¹ to Y⁵ each independently represent the monovalent substituent Ms, a hydrogen atom, a fluorine atom, or a monovalent aliphatic group having no fluorine atom (but excluding monovalent groups containing a saturated hydrocarbon group), and one or two of the combinations of Y¹ and Y², Y² and Y³, Y³ and Y⁴, and Y⁴ and Y⁵ are optionally bonded together to form a ring together with carbon atoms bonded thereto, provided that at least one of Y¹ to Y⁵ represents the monovalent substituent Ms.

10. The compound according to Claim 9, wherein at least one of Y¹ to Y⁵ represents a monovalent group containing a fluorine-containing aliphatic group.

11. The compound according to Claim 9, wherein
m in the formula 1 represents 2, and A represents a group represented by formula A-1a shown below, or
m in the formula 1 represents 3, and A represents a group represented by formula A-2a shown below, or
m in the formula 1 represents 4, and A represents a group represented by formula A-3a shown below: 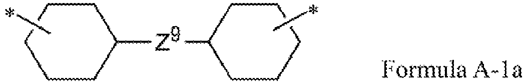 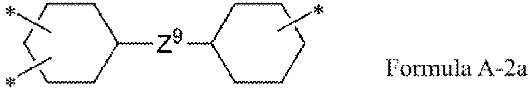 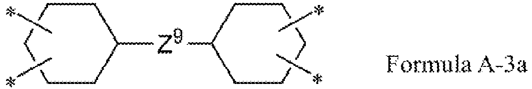 wherein Z⁹ represents a single bond or a divalent linking group that does not contain an aromatic ring structure, and * represents a bonding site.

12. A conductive film having a first region, and a second region that exhibits a higher light transmittance than that of the first region, wherein
the conductive film has a first film containing a conductive material, and a second film containing the compound according to any one of Claims 1 to 11, and
the first film is disposed so as to overlap at least the first region from among the first region and the second region, and the second film is disposed so as to overlap the second region.

13. The conductive film according to Claim 12, wherein the conductive material comprises silver.

14. A photoelectronic device having the conductive film according to Claim 12.

15. The photoelectronic device according to Claim 14, having
a substrate,
an anode provided on the substrate,
a cathode provided on the substrate, and
an active layer disposed between the anode and the cathode, wherein
the cathode is the conductive film.

16. A method for producing a conductive film, the method comprising:
depositing the compound according to any one of Claims 1 to 11 on a substrate via a mask to form a second film containing the compound, and
depositing a conductive material from above the second film.

17. The method for producing a conductive film according to Claim 16, wherein the conductive material comprises silver.
